(19) Europäisches Patentamt
European Patent Office
Office européen des brevets

(11) **EP 1 683 476 B1**

(12) # EUROPEAN PATENT SPECIFICATION

(45) Date of publication and mention
of the grant of the patent:
**08.06.2011 Bulletin 2011/23**

(51) Int Cl.:
***A61B 5/145*** (2006.01)

(21) Application number: **06001000.6**

(22) Date of filing: **18.01.2006**

(54) **Analyte extracting cartridge set**

Set mit einer Kassette zum Extrahieren von Analyten

Kit avec cartouche permettant l'extraction d'analytes

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **19.01.2005 JP 2005011355
17.10.2005 JP 2005301277**

(43) Date of publication of application:
**26.07.2006 Bulletin 2006/30**

(73) Proprietor: **SYSMEX CORPORATION
Kobe-shi,
Hyogo 651-0073 (JP)**

(72) Inventors:
• **Sawa, Kenichi
c/o Sysmex Corporation
Chuo-ku
Kobe-shi
Hyogo 651-0073 (JP)**

• **Maekawa, Yasunori
c/o Sysmex Corporation
Chuo-ku
Kobe-shi
Hyogo 651-0073 (JP)**
• **Asakura, Yoshihiro
c/o Sysmex Corporation
Chuo-ku
Kobe-shi
Hyogo 651-0073 (JP)**

(74) Representative: **HOFFMANN EITLE
Patent- und Rechtsanwälte
Arabellastraße 4
81925 München (DE)**

(56) References cited:
**EP-A- 1 561 418 WO-A-97/02811
US-B1- 6 503 198**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to an analyte extracting cartridge set and a method for loading an analyte extracting cartridge onto an analyzing device.

BACKGROUND

Conventionally, a body fluid extracting apparatus for extracting a body fluid through feeding the skin with an electric current has been known (for example, as disclosed in International Patent Publication No. WO96/00110). The body fluid extracting apparatus disclosed in No. WO96/00110 is arranged in which the body fluid extracted is subjected to an electrically conductive medium such as a hydro-gel stored in a collector reservoir and its contained analyte to be analyzed is measured by a sensor(s) mounted to the collector reservoir for analysis. It is also proposed in International Patent Publication No. WO96/00110 to stabilize the components in the electrically conductive medium through adding a dehydrated gel or dried form of the conductive medium with water or electrolytic solution before exposed to the skin to be examined.

However, the body fluid extracting apparatus disclosed in International Patent Publication No. WO96/00110 using a hydro-gel form of the electrically conductive medium fails to store the hydro-gel for an extensive period of time because water in the hydro-gel in the collector reservoir is highly evaporable.

Also, when the electrically conductive medium is used as a dehydrated gel in a dried form, the body fluid extracting apparatus disclosed in International Patent Publication No. WO96/00110 may have difficulty in adding the conductive medium with water. If the addition of water is too small, it will interrupt the extracting action. If too much water is added, its abundance may injure the body fluid extracting apparatus without being successfully absorbed by the dehydrated gel. Accordingly, the body fluid extracting apparatus will be difficult in the handling.

Reference is also directed to US Patent No. US 6,503,198 B1, which discloses noninvasive transdermal patches comprised of a wet chemistry component and a dry chemistry component. The wet chemistry component is a liquid transfer medium in the form of a gel layer for the extraction and liquid bridge transfer of the analyte of interest from the biological fluid within or beneath the skin to the dry chemistry component. The dry chemistry component is a super sensitive or conditioned membrane carrying a reagent system for interacting with the analyte of interest to generate an indicator molecule, e.g., color change, to confirm detection of the analyte. A configuration is described which is specifically designed for keeping the dry chemistry component and the wet chemistry component intact and separate from one another during non-use, but which allows them to intimately engage one another during testing, so that the dry chemistry component is continuously and uniformly wetted during testing by the wet chemistry component and the analyte under investigation can be extracted and transferred from the biological fluid within or beneath the skin to the super sensitive or concentrated member for interaction with the chemical reagents to generate the indicator molecules.

International application WO 97/02811 discloses a patch comprised of a hydrophilic compound which forms a material which holds water in place and allows the flow of electrical current therethrough. The compound may be an absorbent material, porous material or polymers which may be cross-linked to form a porous network of interconnected cells or a solute which forms a gel with water. The patch comprises an enzyme, which catalyzes a reaction such as a reaction with glucose allowing for the formation of hydrogen peroxide in water and ultimately generating the release of two electrons per molecule of glucose. Glucose drawn into the patch is reduced to gluconic acid and hydrogen peroxide with the aid of the enzyme and in use resulting in electrons being released which can be detected and related to the amount of glucose entering the patch. The patch is preferably in the form of a thin, flat disc which will conform to the contours of human skin and may have non-woven fabric or porous membrane embedded therein.

SUMMARY

The scope of the present invention is defined solely by the appended claims, and is not affected to any degree by the statements within this summary.

According to a first aspect of the present invention, there is provided a method for loading an analyte extracting cartridge onto an analyzing device which is arranged to extract an analyte from a subject to be examined and analyze the analyte, comprising steps of: (a) supplying a liquid from a liquid distributor member which retains the liquid to a liquid retaining member in the analyte extracting cartridge which can retain the liquid for holding the analyte extracted from the subject, the analyte extracting cartridge and the liquid distributor member being held by a holding member; (b) separating the analyte extracting cartridge from the liquid distributor member and holding member after distributing the liquid to the liquid retaining member; and (c) loading the analyte extracting cartridge onto the analyzing device.

According to a second aspect of the present invention, there is provided an analyte extracting cartridge set comprising: an analyte extracting cartridge to be loaded to an analyzing device for extracting an analyte from a subject to be examined and analyzing the analyte, the analyte extracting cartridge having a liquid retaining member for retaining a liquid to hold the analyte extracted from the subject; a liquid distributor member for retaining the liquid and distributing the liquid to the liquid retaining member, wherein the liquid distributor member is separable from the analyte extracting cartridge, so that it may be separated therefrom after distributing the liquid to the liquid retaining member; a holding member for holding the analyte extracting cartridge and the liquid distributor member, the analyte extracting cartridge being detachably bonded to the holding member; and a separator member for isolating the liquid retaining member in the analyte extracting cartridge from the liquid distributor member.

There is also described in the following for explanatory purposes only a method not constituting an embodiment of the present invention, the method being for extracting an analyte from a subject to be examined and analyzing the analyte with the use of an analyte extracting cartridge to be loaded to an analyzing apparatus, the method including: (a) supplying a liquid from a liquid distributor member which retains the liquid to a liquid retaining member in the analyte extracting cartridge which can retain the liquid for holding the analyte extracted from the subject; (b) loading the analyte extracting cartridge onto the analyzing apparatus; (c) mounting the analyzing apparatus loaded with the analyte extracting cartridge to the subject; (d) extracting the analyte from the subject into the liquid carried in the liquid retaining member; and (e) analyzing the analyte extracted into the liquid.

An analyte extracting cartridge set described herein includes: an analyte extracting cartridge to be loaded to an analyzing apparatus for extracting an analyte from a subject to be examined and analyzing the analyte, the analyte extracting cartridge having a liquid retaining member for retaining a liquid to hold the analyte extracted from the subject; and a liquid distributor member for retaining the liquid and distributing the liquid to the liquid retaining member.

A second analyte extracting cartridge set described herein includes: an analyte extracting cartridge to be loaded to an analyzing apparatus for extracting an analyte from a subject to be examined and analyzing the analyte, the analyte extracting cartridge having a liquid retaining member for retaining a liquid to hold the analyte extracted from the subject; a first film member detachably bonded to a surface of the analyte extracting cartridge; a liquid distributor member for retaining the liquid and distributing the liquid to the liquid retaining member; a liquid distributor member holding member having an inner surface facing the surface of the analyte extracting cartridge for holding the liquid distributor member; a second film member detachably bonded to the inner surface of the liquid distributor member holding member defining a space with the liquid distributor member holding member for holding the liquid distributor member; and a joining member joined to one end of the first film member and one end of the second film member, wherein when the joining member is urged in one direction by a force, the first film member and the second film member are detached from the surface of the analyte extracting cartridge and the inner surface of the liquid distributor member holding member respectively.

A third analyte extracting cartridge set described herein includes: an analyte extracting cartridge to be loaded to an analyzing apparatus for extracting an analyte from a subject to be examined and analyzing the analyte, the analyte extracting cartridge having a liquid retaining member for retaining a liquid to hold the analyte extracted from the subject; and a container for enclosing the liquid to be distributed to the liquid retaining member.

BRIEF DESCRIPTION OF THE DRAWINGS

Fig. 1 is a perspective view of a blood sugar level measuring apparatus loaded with an extracting cartridge according to a first embodiment of the present invention and mounted to the wrist of a subject to be examined;
Fig. 2 is a plan view of an internal arrangement of the blood sugar level measuring apparatus shown in Fig. 1;
Fig. 3 is a cross sectional view taken along the line 300-300 of Fig. 2;
Fig. 4 is a cross sectional view taken along the line 400-400 of Fig. 2;
Fig. 5 is an enlarged view of the blood sugar level measuring apparatus shown in Fig. 3 mounted to the wrist of the subject to be examined;
Fig. 6 is a plan view of a construction of the extracting cartridge loaded to the blood sugar level measuring apparatus shown in Fig. 2;
Fig. 7 is a schematic view showing a detector in the blood sugar level measuring apparatus shown in Fig. 1;
Fig. 8 is a perspective view of the extracting cartridge of the first embodiment;
Fig. 9 is a cross sectional view taken along the line 500-500 of Fig. 8;
Fig. 10 is a perspective view of the extracting cartridge of the first embodiment, shown in Fig. 8, in an open state;
Fig. 11 is a plan view of the extracting cartridge of the first embodiment shown in Fig. 10;
Fig. 12 is a perspective view of micro needles used in a preparatory step;
Fig. 13 is a cross sectional view of the skin subjected to the preparatory step with the micro needles shown in Fig. 12;
Fig. 14 is a flowchart showing a procedure for measuring the blood sugar level;
Figs. 15 to 18 are cross sectional views explaining the action of taking out the extracting cartridge from an extracting cartridge set of the first embodiment shown in Fig. 8;

Fig. 19 is a perspective view explaining an action of taking out the extracting cartridge from an extracting cartridge set of the first embodiment shown in Fig. 8;

Fig. 20 is a profile showing the relationship between the storage of pure water in a liquid distributor member in the extracting cartridge set of the first embodiment shown in Fig. 8 and the distributed amount of pure water from the liquid distributor member to a mesh sheet in the extracting cartridge;

Figs. 21 to 24 are schematic views illustrating the principle of extracting glucose with the use of the blood sugar level measuring apparatus shown in Fig. 1;

Fig. 25 is a plan view of an internal arrangement of the blood sugar level measuring apparatus loaded with a modification of the extracting cartridge of the first embodiment;

Fig. 26 is a perspective view of an extracting cartridge set according to a second embodiment of the present invention;

Fig. 27 is across sectional view taken along the line 600-600 of Fig. 26;

Fig. 28 is a cross sectional view taken along the line 700-700 of Fig. 26;

Fig. 29 is a perspective view of a separator member in the extracting cartridge set of the second embodiment of the present invention;

Figs. 30, 32, 35, 37, and 39 are perspective views showing the arrangement and action of separating the separator member from the extracting cartridge set of the second embodiment;

Figs. 31, 33, 36, 38, and 40 are cross sectional views showing the arrangement and action of separating the separator member from the extracting cartridge set of the second embodiment;

Fig. 34 is an enlarged cross sectional view showing the arrangement and action of separating the separator member from the extracting cartridge set of the second embodiment;

Fig. 41 is a perspective view showing the arrangement and action of distributing the liquid from a liquid distributor member to a liquid retaining member in the second embodiment;

Fig. 42 is a cross sectional view showing the arrangement and action of distributing the liquid from the liquid distributor member to the liquid retaining member in the second embodiment;

Fig. 43 is a perspective view showing the arrangement and action of taking out a housing member from the extracting cartridge set of the second embodiment;

Fig. 44 is a cross sectional view showing the arrangement and action of taking out a housing member from the extracting cartridge set of the second embodiment; and

Fig. 45 is a diagram showing the fusing characteristics of a PE (polyethylene) based film of a cartridge base in the second embodiment.

## DETAILED DESCRIPTION OF THE INVENTION

Some embodiments of the present invention will be described referring to the relevant drawings.

(First Embodiment)

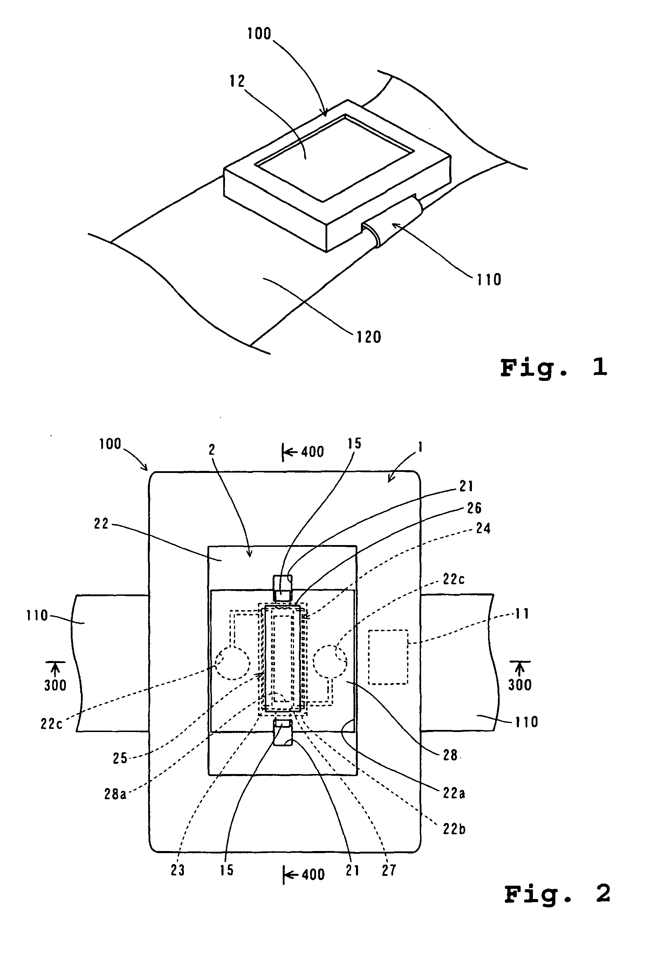

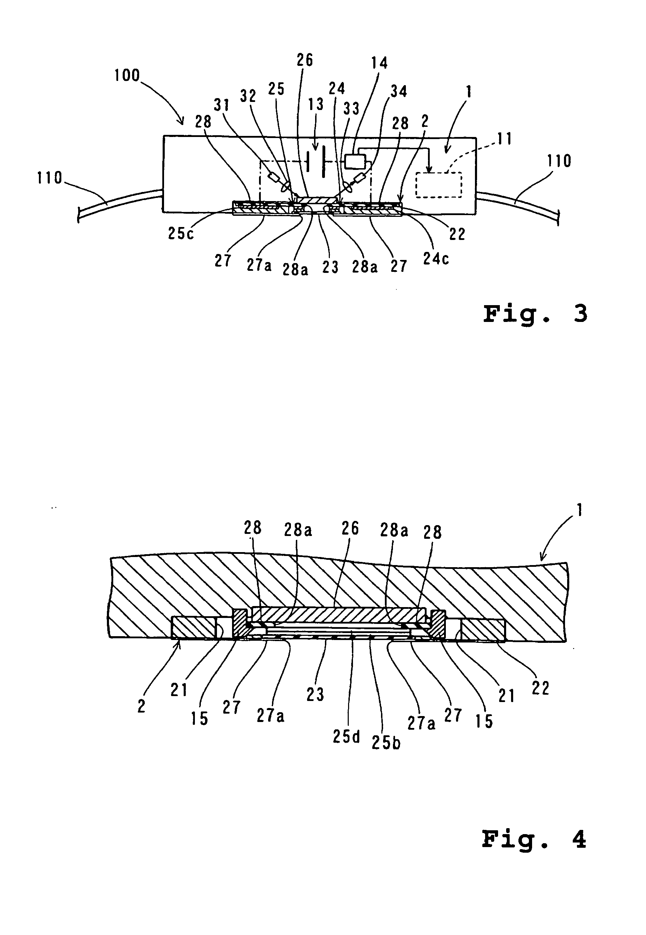

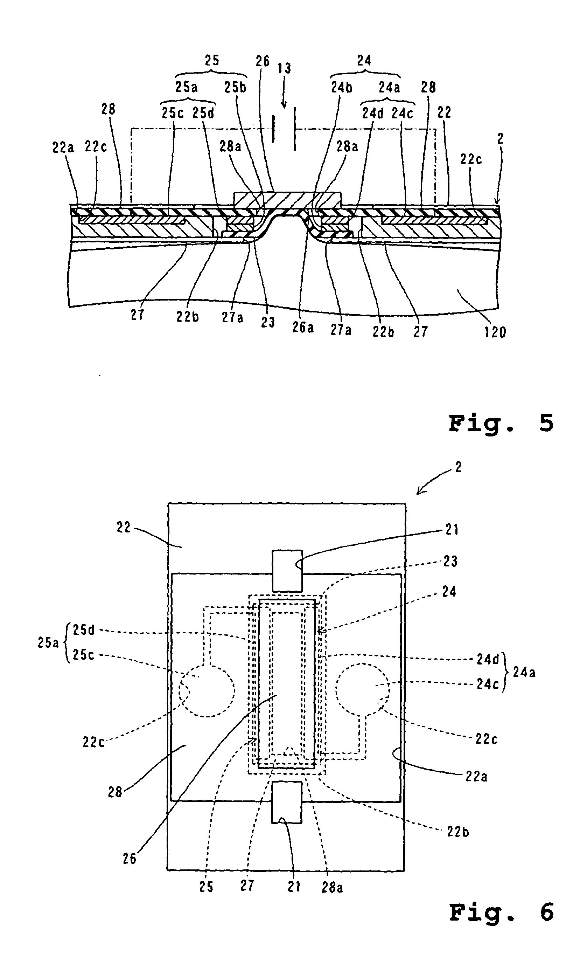

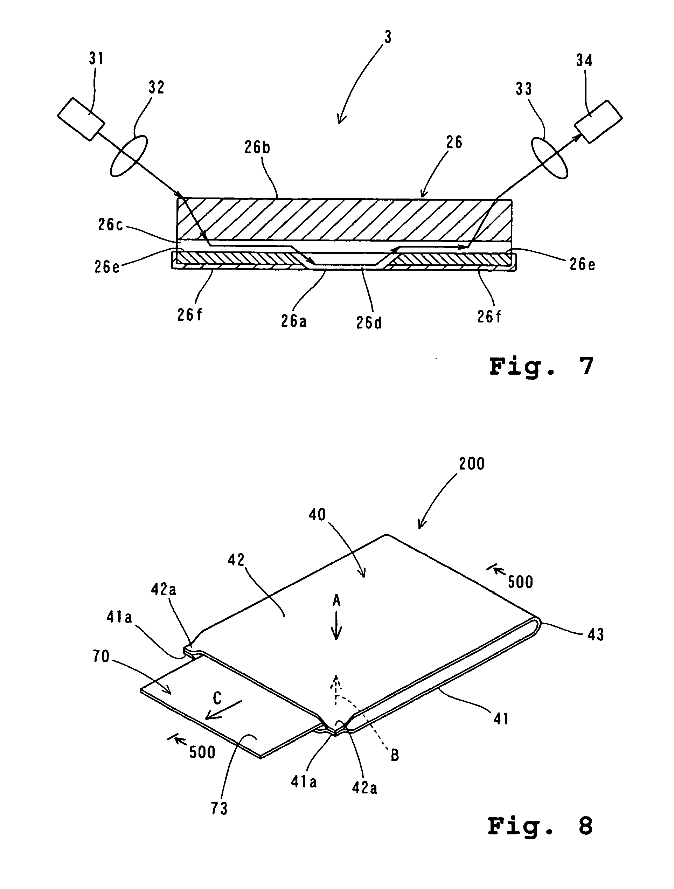

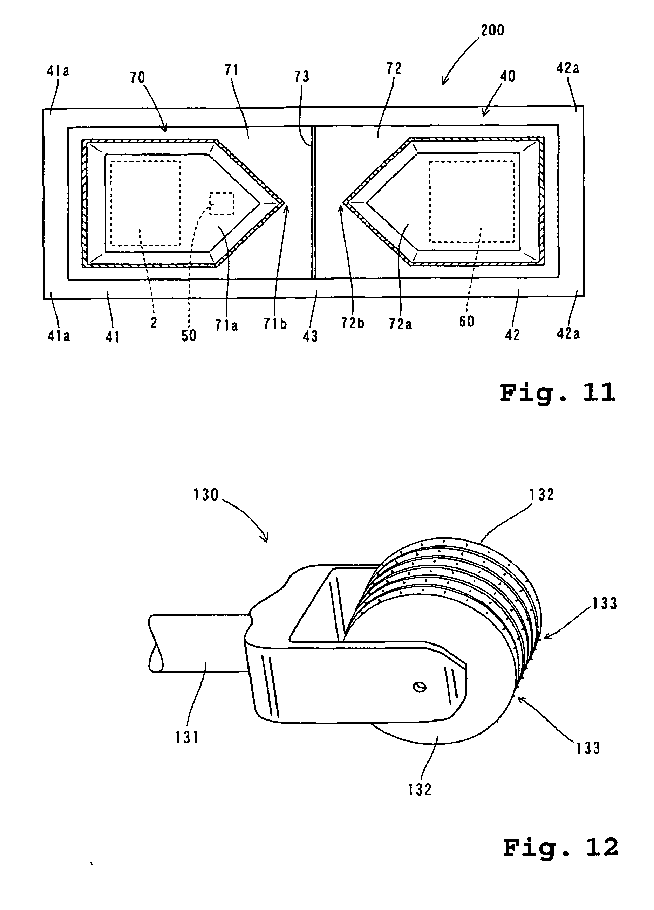

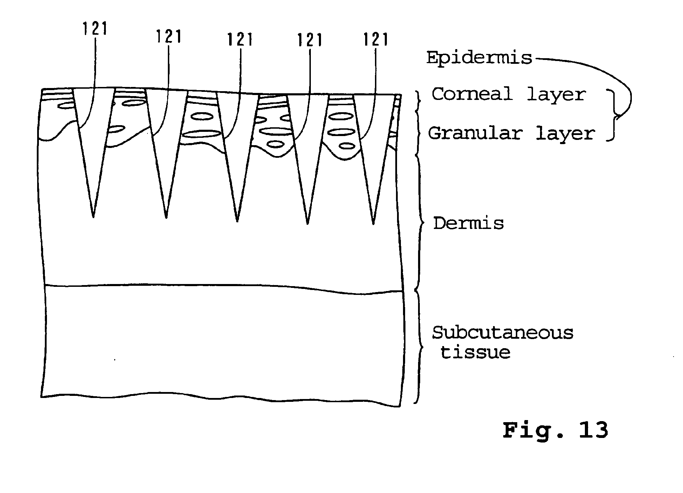

Fig. 1 is a perspective view of a blood sugar level measuring apparatus loaded with an extracting cartridge according to the first embodiment of the present invention and mounted to the wrist of a subject. Figs. 2 to 4 illustrate an internal arrangement of the blood sugar level measuring apparatus shown in Fig. 1. Fig. 5 is an enlarged view of the blood sugar level measuring apparatus, shown in Fig. 3, mounted to the wrist of a subject. Fig. 6 is a plan view of a construction of the extracting cartridge, shown in Fig. 2, to be loaded onto the blood sugar level measuring apparatus. Fig. 7 is a schematic view of a detector in the blood sugar level measuring apparatus shown in Fig. 1. Figs. 8 to 11 are perspective views of an extracting cartridge set in the first embodiment. Fig. 12 is a perspective view of micro-needles used in the preparation step. Fig. 13 is a cross sectional view of a skin part subjected to the preparation step with the micro-needles shown in Fig. 12, which step is described herein for explanatory purposes only and does not form part of the invention as claimed. The entire arrangement of the blood sugar level measuring apparatus 100 loaded with the extracting cartridge 2 in the first embodiment will be described referring to Figs. 1 to 7, Fig. 12, and Fig. 13.

The blood sugar level measuring apparatus 100 is designed for extracting glucose as a biochemical compound from a living body and analyzing the same to calculate the blood sugar level. The blood sugar level measuring apparatus 100 is equipped with a band member 110 for mounting to the wrist 120 of a subject, as shown in Fig. 1. The band member 110 has a fitting (not shown) provided with an opening at a specific location. The opening in the fitting allows the subject to operate a needle roller 130 (See Fig. 12) for conducting a preparation action on a target area of the wrist 120 of the subject. The needle roller 130 does not form part of the present invention and its following description is included for explanatory purposes only.

The needle roller 130 comprises, as shown in Fig. 12, an arm 131 and a group of rollers 132 rotatably supported by the arm 131. There is a plurality of small needles 133 arranged at equal intervals on the outer surface of each roller 132. The needles 133 are sized (about 0.3 mm in length) not to reach the subcutaneous tissue but enough to penetrate through the epidermis including a corneal layer. In action, the needle roller 130 produces extracting apertures 121 which extend across the epidermis to the dermis but not to the subcutaneous tissue, as shown in Fig. 13, allowing the humor to be extracted through the apertures 121 from the body. This can lessen the pain perceived by the subject when the blood sugar level measuring apparatus 100 shown in Fig. 1 is used for extracting glucose from the body. The needle roller 130 in the first embodiment may be a Derma roller made by Top-Rol.

As shown in Figs. 2 and 3, the blood sugar level measuring apparatus 100 includes an analyzer unit 1 as a main body of the apparatus, and an extracting cartridge 2 detachably held in the analyzer unit 1. The analyzer unit 1 comprises, as shown in Figs. 2 and 3, a controller 11, a display 12 (See Fig. 1), a constant DC voltage source 13 (See Fig. 3), a current meter 14 (See Fig. 3) for measuring the current from the constant voltage source 13 and feeding the controller 11 with its measurement, and a pair of engaging hooks 15. The analyzer unit 1 includes a monochromatic light source 31, a couple of lenses 32 and 33, and a light receiving element 34 which constitute a detector 3 shown in Fig. 7. The controller 11 is composed mainly of a CPU, a ROM, and a RAM for calculating the blood sugar level from the measurement of glucose level. The display 12 is provided for displaying the measurement of glucose level and the blood sugar level calculated by the controller 11.

The constant voltage source 13 is connected, as shown in Figs. 3 and 5, with the terminal port 24c of an anode 24 and the terminal port 25c of a cathode 25 in the extracting cartridge 2 which will be explained later. Impressed between the anode 24 and the cathode 25 is a voltage of 0.8 V from the constant voltage source 13. The two engaging hooks 15 are arranged for securing the extracting cartridge 2 to the analyzer unit 1. The monochromatic light source 31 and the lens 32 in the detector 3 of the analyzer unit 1 are provided for supplying a sensor member 26, which will be explained later, with analyzing illumination as shown in Figs. 3 and 7. In action, the light is passed through the sensor member 26 and received via the lens 33 by the light receiving element 34.

More particularly, the extracting cartridge 2 is, as shown in Figs. 2 and 4, detachably secured to the analyzer unit 1 with its two engaging holes 21 receiving the engaging hooks 15 of the analyzer unit 1 respectively. The extracting cartridge 2 is arranged exchangeable from one measurement of the blood sugar level to another.

In the first embodiment, the extracting cartridge 2 comprises, as shown in Figs. 5 and 6, a cartridge base 22 made of an acrylic resin material, a mesh sheet 23 provided as a medium for carrying pure water, the anode 24 and the cathode 25, the sensor member 26 provided as a primary component in the detector 3 (See Fig. 7) for detecting the measurement of glucose level, a double-coated tape 27, and an electrode sheet 28 made of substantially an insulating material. The cartridge base 22 has a stepped portion 22a arranged of a four-surfaced shape as shown in Fig. 6. The cartridge base 22 also has a through opening 22b provided in the center of the stepped portion 22a thereof to extend down to the lower surface thereof as shown in Fig. 5. A pair of circular recesses 22c are provided at both surfaces of the center opening 22b in the stepped portion 22a of the cartridge base 22. The two circular recesses 22c are shaped to accept the terminal port 24c of the anode 24 and the terminal port 25c of the cathode 25 respectively. The mesh sheet 23 with a pair of active carbon electrodes 24b and 25b and operating strips 24d and 25d are accommodated in the center opening 22b. The electrode sheet 28 is fitted into the stepped portion 22a of the cartridge base 22 as sandwiched between the sensor member 26 and the operating strips 24d and 25d. The electrode sheet 28 has an opening 28a provided therein to measure substantially 9 mm by 3 mm. The measuring surface 26a of the sensor member 26 is exposed across the opening 28a in the electrode sheet 28 to the surface of the mesh sheet 23.

In the first embodiment, the mesh sheet 23 is set in direct contact with the lower surfaces of the active carbon electrodes 24b and 25b and fixedly supported from below of the cartridge base 22 by the double-coated tape 27 of about 20 μm thick, as shown in Fig. 5. The double-coated tape 27 has an opening 27a provided therein for defining the measuring area (extracting area) of the skin. The cartridge base 22 is entirely covered at the lower surface with the double-coated tape 27. At the time of glucose extraction, the mesh sheet 23 is impregnated with pure water. This allows the mesh sheet 23 to be lifted up to extend across the opening 27a of the double-coated tape 27 and come into direct contact with the lower surface of the sensor member 26 by the wrist 120 of the subject pressing upwardly at the skin. The mesh sheet 23 in the first embodiment is made of a nylon material, which is substantially 10 mm in length, 4 mm in width, and 50 μm in thickness. More precisely, the thickness of the mesh sheet 23 may range substantially from 1 μm to 1 mm (1000 μm) or preferably from 30 μm to 1 mm (1000 μm) . Since the mesh sheet 23 is not smaller than 30 μm in the thickness, it will surely be prevented from being injured by the skin pressing up from below during the measuring action.

As the mesh sheet 23 is not greater than 1 mm (1000 μm) in the thickness, it can contribute to the reduction of the time required for glucose extracted from the skin deep and reaching to the measuring surface 26a of the sensor member 26, as compared with the case the thickness of greater than 1 mm (1000 μm) is used. As the result, the duration of time from the start of extracting glucose and measuring the extracted amount of glucose with the sensor member 26 can be minimized. In other words, the process of determining the measurement of glucose level and calculating the blood sugar level can be shortened. The mesh sheet 23 is arranged flexible but substantially not elastic for shrinkage or contraction thus to remain unchanged in the thickness. The mesh sheet 23 is a square woven form, 33 μm by 33 μm,

of nylon fiber of about 30 μm thick. This allows the mesh sheet 23 to be highly porous as woven. The distance between the skin at the wrist 120 of the subject and the two electrodes 24 and 25 is arranged substantially equal to the thickness of the mesh sheet 23 and set to 50 μm in the first embodiment.

The anode 24 and the cathode 25 are provided with the electrode collectors 24a and 25a made of silver chloride (AgCl) and the active carbon electrodes 24b and 25b made of a porous electrically conductive material or active carbon material, as shown in Figs. 5 and 7. The electrode collectors 24a and 25a consist of the ports 24c and 25c fitted into the circular recesses 22c in the stepped portion 22a of the cartridge base 22 and the operating strips 24d and 25d connected with the ports 24c and 25c respectively. The active carbon electrodes 24b and 25b are bonded to the lower surfaces of the operating strips 24d and 25d of the collectors 24a and 25a respectively while their specific area ranges substantially from 1000 $m^2$/g to 3000 $m^2$/g. Both the terminal port 24c of the anode 24 and the terminal port 25c of the cathode 25 are connected to the constant DC voltage source 13 provided in the analyzer unit 1.

Referring to Fig. 5, the sensor member 26 is disposed on the upper surface of the electrode sheet 28 and its measuring surface 26a is arranged for measuring the extracted amount of glucose. The measuring surface 26a is coated with a gel mixture of a coloring agent for generating a color upon reacting with glucose and a particular enzyme before dried out.

More specifically, the gel mixture applied to the measuring surface 26a of the sensor member 26 before dried out contains glucose oxydase (GOD) which is an oxidizing enzyme acting as a catalyst for glucose, per-oxydase (POD) which is an oxidizing and reducing enzyme acting as a catalyst for hydrogen peroxide ($H_2O_2$) produced by the catalytic reaction between glucose and GOD, and a coloring agent for generating a color upon reacting with active oxygen (0*) produced by the catalytic reaction between $H_2O_2$ and POD.

The sensor member 26 comprises, as shown in Fig. 7, a glass substrate 26b, a first optical waveguide layer 26c disposed beneath the substrate 26b, a second optical waveguide layer 26d disposed at the center beneath the first optical waveguide layer 26c, a protective layer 26e disposed beneath the first optical waveguide layer 26c to sandwich the second optical waveguide layer 26d with the first optical waveguide layer 26c, and a shielding layer 26f covering over the outer surface of the protective layer 26e. The first optical waveguide layer 26c is higher in the index of refraction than the substrate 26b. The second optical waveguide layer 26d is arranged of a trapezoidal shape beveled at surfaces and is higher in the index of refraction than the first optical waveguide layer 26c. The measuring surface 26a of the sensor member 26 is exposed from the protective layer 26e over the second optical waveguide layer 26d so as to come into direct contact with the upper surface of the mesh sheet 23 in the measuring action, as shown in Fig. 5. The sensor member 26 is combined with the monochromatic light source 31, the two lenses 32 and 33, and the light receiving element 34 in the analyzer unit 1 thus to constitute the detector 3 in the first embodiment.

The arrangement of an extracting cartridge set 200 for accommodating an unused extracting cartridge 2 to be loaded onto the blood sugar level measuring apparatus 100 will be described referring to Figs. 3, 4, and 6 and Figs. 8 to 11.

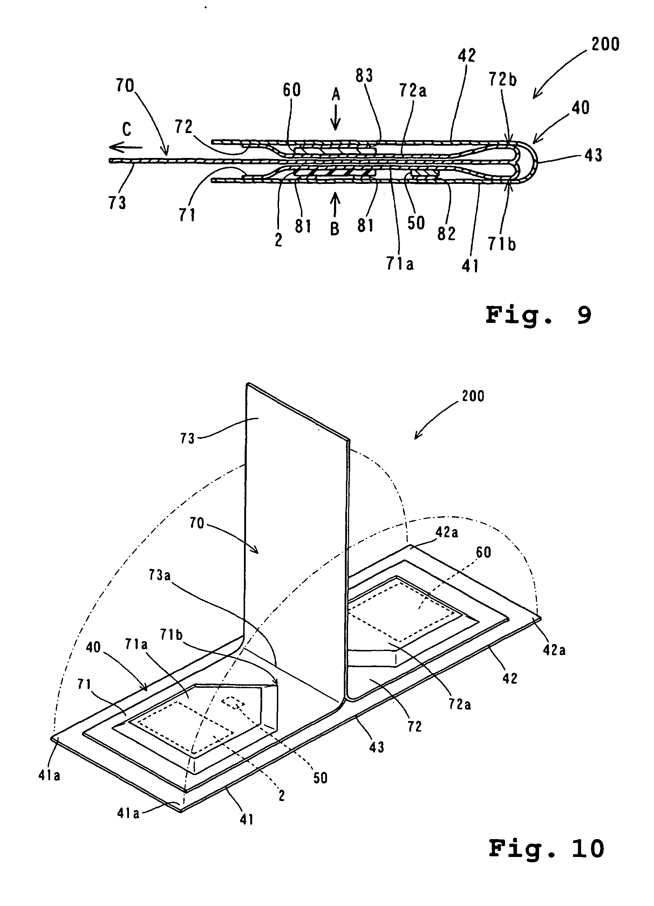

The extracting cartridge set 200 in the first embodiment is designed for accommodating an unused extracting cartridge 2 in its dry condition before loaded onto the blood sugar level measuring apparatus 100 and capable of allowing the mesh sheet 23 in the unused extracting cartridge 2 to absorb a predetermined amount of pure water just before loaded to the blood sugar level measuring apparatus 100. The extracting cartridge set 200 in the first embodiment comprises, as shown in Figs. 8 and 9, a support member 40, the unused extracting cartridge 2 (See Fig. 9), a desiccant 50 (See Fig. 9), a liquid distributor member 60 (See Fig. 9), and a separator member 70.

The support member 40 is a sheet of flexible material. The support member 40 has a three-layer construction including a PET (polyethylene terephthalate) layer, an aluminum foil layer, and a polyethylene layer laminated in this order. The support member 40 is bent to be in a U-shape with its polyethylene layer surface disposed at the inside. The support member 40 comprises a cartridge support 41, a liquid distributor member support 42, disposed opposite to the cartridge support 41, and a bent 43 for coupling between the cartridge support 41 and the liquid distributor member support 42. As shown in Fig. 8, the cartridge support 41 has two corners 41a thereof joined to two corresponding corners 42a of the liquid distributor member support 42 by fusing the polyethylene layers at each of the corners 41a and 42a. As the support member 40 is arranged to practically be in a pouch shape, it allows the unused extracting cartridge 2 and the liquid distributor member 60 to be held (accommodated) in a space defined by the cartridge support 41, the liquid distributor member support 42, and the bent 43. The fusing between the corners 41a of the cartridge support 41 and the corners 42a of the liquid distributor member support 42 provides a bonding strength, which can easily be released.

As shown in Fig. 9, the extracting cartridge 2 is detachably bonded by a pair of soft double-coated tapes 81 to the inner surface of the cartridge support 41. Also, the extracting cartridge 2 is oriented with its mesh sheet 23 facing at the skin surface the liquid distributor member 60 (upwardly in Fig. 9).

The desiccator 50 is spaced by a distance from the extracting cartridge 2 as tightly bonded by an adhesive 82 to the inner surface of the cartridge support 41 of the support member 40. The desiccator 50 is provided for inhibiting the mesh sheet 23 (See Fig. 6) of the extracting cartridge 2 from absorbing water in the air and becoming damp.

The liquid distributor member 60 is a size of unwoven fabric such as absorbent cotton (cut cotton), which is substantially 15 mm in length, 15 mm in width, and 50 μm in thickness. The liquid distributor member 60 carries a predetermined amount (about 150 μl in the first embodiment) of pure water. The pure water in the first embodiment has an electrical resistivity (specific resistivity) of 18.3 MΩ·cm and is thus substantially an insulating substance (a non-conductive material). As shown in Fig. 9, the liquid distributor member 60 is bonded by an adhesive 83 to the inner surface of the liquid distributor member support 42 of the support member 40 so as to oppose to the mesh sheet 23 of the extracting cartridge 2 bonded to the cartridge support 41 of the support member 40. Since the liquid distributor member 60 (substantially 15 mm by 15 mm) is greater in the size than the opening 28a (substantially 9 mm by 3 mm) in the electrode sheet 28 disposed on the measuring surface 26a of the sensor member 26 in the extracting cartridge 2 and the liquid distributing member 60, its distribution of pure water to the mesh sheet 23 of the extracting cartridge 2 can be guaranteed even if its position is slightly dislocated in relation to the extracting cartridge 2. In the first embodiment, the pure water (150 μl) carried in the liquid distributor member 60 is distributed about one percent (1.5 μl) at each time to the mesh sheet 23 of the extracting cartridge 2. As the mesh sheet 23 is constantly supplied with a given amount of the pure water from the liquid distributor member 60, its moisture level remains unchanged regardless of more or less evaporation of the pure water in the liquid distributor member 60, thus increasing the storage period of the extracting cartridge 2.

Similar to the support member 40, the separator member 70 is a sheet of flexible material, which has a three-layer construction including a PET (poly ethylene terephthalate) layer at the innermost surface, an aluminum foil layer, and a polyethylene layer at the outermost surface. The separator member 70 is accommodated, as shown in Figs. 8 and 9, in a space defined by the cartridge support 41, the liquid distributor member support 42, and the bent 43 of the U shaped support member 40. The separator member 70 is bonded by thermally fusing at a predetermined location of its outer surface or polyethylene layer to the inner polyethylene layer of the support member 40. As shown in Fig. 9, the separator member 70 comprises a cartridge holder 71 arranged opposite to the cartridge support 41 of the support member 40, a liquid distributor member holder 72 arranged opposite to the liquid distributor member support 42 of the support member 40, and an insertion 73 sandwiched between the cartridge holder 71 and the liquid distributor member holder 72. The cartridge holder 71 and the liquid distributor member holder 72 are formed integral with the insertion 73 as extending from a predetermined end 73a of the insertion 73 in opposite directions, as shown in Fig. 10.

As shown in Figs. 9, 10, and 11, the cartridge holder 71 has a recessed portion 71a thereof shaped for accepting the extracting cartridge 2 and the desiccant 50 on the cartridge support 41 of the support member 40. The recessed portion 71a of the cartridge holder 71 is arranged of a pentagonal home-base shape in the plan view. Also as shown in Fig. 11, the cartridge holder 71 is bonded by thermally fusing at a periphery of its outer surface or polyethylene layer, which surrounds the recessed portion 71a, to the inner polyethylene layer of the support member 40. This allows the extracting cartridge 2 and the desiccator 50 to be securely held in the enclosed space defined by the cartridge support 41 of the support member 40 and the cartridge holder 71 at the recessed portion 71a of the separator member 70. As the result, the extracting cartridge 2 can securely be stored in its dry condition between the support member 40 and the separator member 70. Also, as the cartridge holder 71 has the recessed portion 71a thereof arranged of the home-base shape, it allows the separator member 70 to be easily separated with a less strength from the support member 40 when having been peeled off by starting from the corner 71b of the recessed portion 71a thereof, as compared with a square shape of the recessed portion 71a.

The liquid distributor member holder 72 also has a recessed portion 72a thereof shaped for accepting the liquid distributor member 60 impregnated with pure water on the liquid distributor member support 42 of the support member 40. The recessedportion 72a of the liquid distributor member holder 72 is arranged of a pentagonal home-base shape in the plan view. Also, the liquid distributor member holder 72 is bonded by thermally fusing at a periphery of its outer surface or polyethylene layer, which surrounds the recessed portion 72a, to the inner polyethylene layer of the support member 40. This allows the liquid distributor member 60 impregnated with pure water to be securely held in the enclosed space defined by the liquid distributor member support 42 of the support member 40 and the liquid distributor member holder 72 at the recessed portion 72a of the separator member 70 . As the liquid distributor member holder 72 has the recessedportion 72a thereof arranged of the home-base shape, it allows the separator member 70 to be easily separated with less strength from the support member 40 when having been peeled off by starting from the corner 72b of the recessedportion 72a thereof, as compared with a square shape of the recessed portion 72a.

The insertion 73 is provided for being pulled by the subject to separate the separator member 70 from the support member 40. As shown in Fig. 9, the insertion 73 extends outwardly with a predetermined length from one end of the support member 40, which is opposite to the bent 43. When the subject holds and pulls an outwardly extended portion of the insertion 73 in the direction denoted by the arrow C, its pulling force is transmitted via the insertion 73 to the cartridge holder 71 and the liquid distributor member holder 72. This allows the cartridge holder 71 and the liquid distributor member holder 72 to be gradually separated from the cartridge holder 41 and the liquid distributor member holder 42 respectively by starting from the corners 71b and 72b of their corresponding recessed portions 71a and 72a.

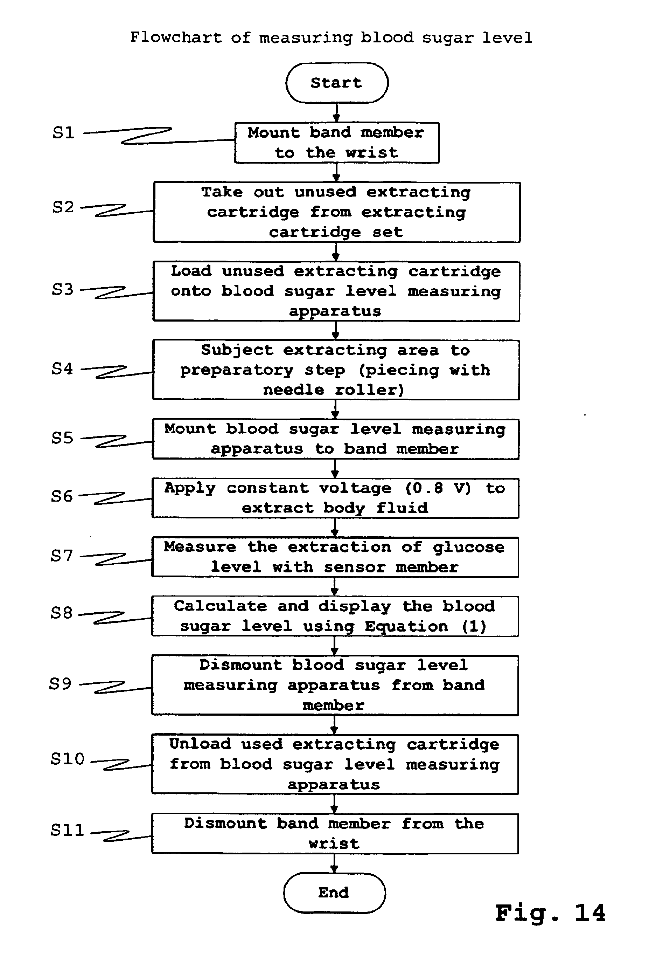

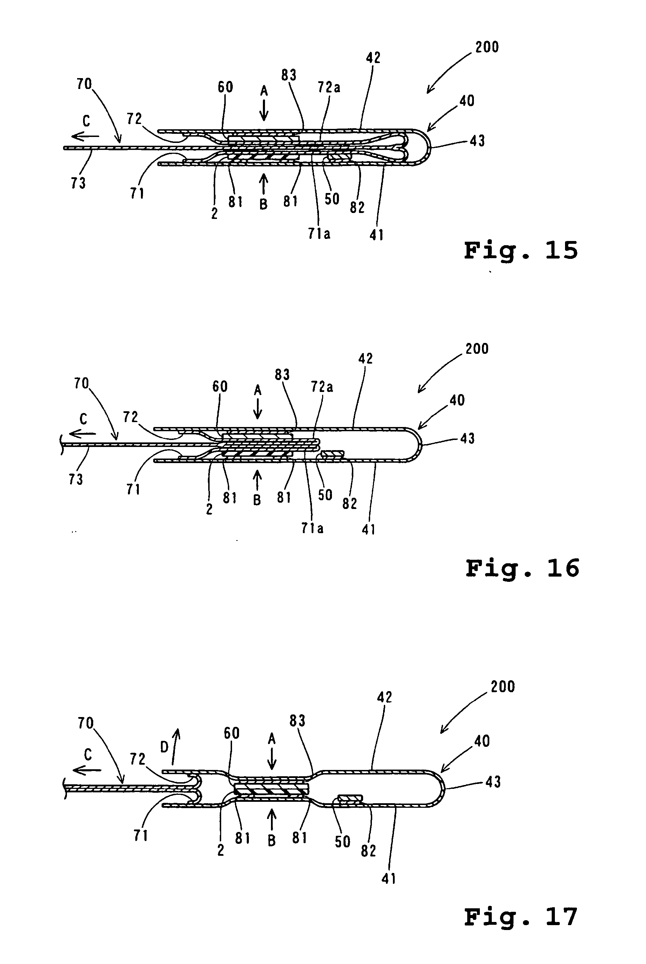

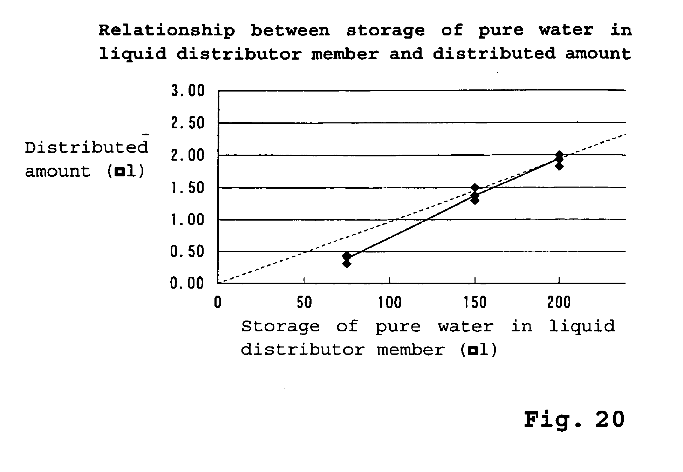

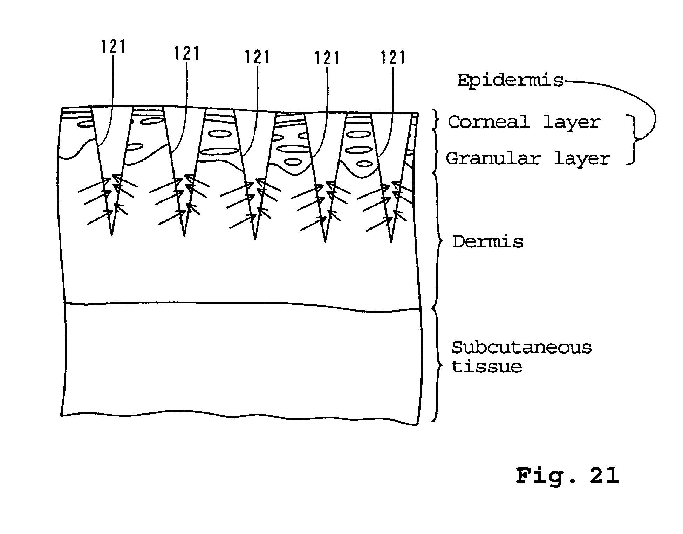

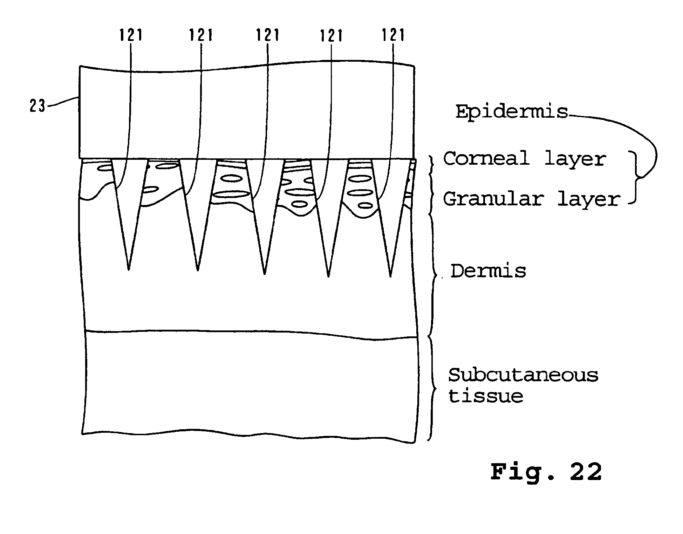

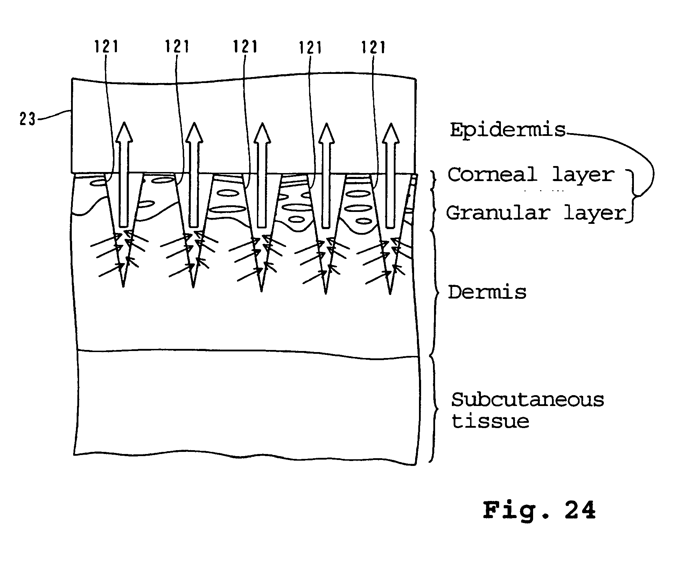

Fig. 14 is a flowchart showing a procedure of the blood sugar level measuring apparatus, shown in Fig. 1, measuring the blood sugar level. Figs. 15 to 19 illustrate steps of taking out the unused extracting cartridge from the extracting cartridge set in the first embodiment shown in Fig. 8. Fig. 20 is a profile showing the relationship between the storage of pure water carried in the liquid distributor member in the extracting cartridge set and the distribution (transfer) of pure water from the liquid distributor member to the mesh sheet in the extracting cartridge. Figs. 21 to 24 are schematic cross sectional views explaining the principle of the glucose extracting action of the blood sugar level measuring apparatus shown in Fig. 1 . The procedure of the blood sugar level measuring apparatus 100 measuring the blood sugar level will now be described referring to Figs. 1, 5, 6, and 9 and Figs. 12 to 24. It is emphasized that the preparatory step shown in Fig. 14 does not form part of the present invention as claimed and that Figs. 21 to 24 and their accompanying description are included for explanatory purposes only.

The procedure starts with Step S1 shown in Fig. 14 for mounting the band member 110 to the wrist 120 of a subject to be examined. More particularly, the band member 110 is mounted so that the opening (not shown) in its fitting locates the measuring area (extracting area) of the wrist 120.

This is followed by Step S2 for the subject taking out an unused extracting cartridge 2 from the extracting cartridge set 200. More specifically, the subject holds the extracting cartridge set 200 with its two fingers pressing from the directions denoted by the arrows A and B respectively against the outer surface side of the cartridge support 41 and the outer surface side of the liquid distributor member support 42 of the support member 40 shown in Figs. 8 and 9. The extracting cartridge set 200 carries the unused dried extracting cartridge 2 and the liquid distributor member 60 impregnated with an amount (150 $\mu$m) of pure water both of which are physically separated from each other as accommodated therein. Then, the subject pulls an exposed portion of the insertion 73 of the separator member 70 in the extracting cartridge set 200 in the direction denoted by the arrow C. This causes the insertion 73 of the separator member 70 to move in the direction denoted by the arrow C, as shown in Figs. 15 and 16. As the insertion 73 of the separator member 70 moves in the direction denoted by the arrow C, the cartridge holder 71 and the liquid distributor holder 72 of the separator member 70 are gradually removed from the cartridge support 41 and the liquid distributor member support 42 of the support member 40 respectively by starting from the corner 71b at the recessed portion 71a of the cartridge holder 71 and the corner 72b at the recessed portion 72a of the liquid distributor member holder 72.

Accordingly as shown in Fig. 17, the separator member 70 is removed between the extracting cartridge 2 in its dry condition and the liquid distributor member 60 impregnated with pure water and also the cartridge support 41 and the liquid distributor member support 42 of the support member 40 are depressed both from the directions denoted by the arrows A and B by the fingers of the subject. This causes the liquid distributor member 60 impregnated with pure water to come into direct contact with the mesh sheet 23 of the extracting cartridge 2, whereby about one percent (substantially 1.5 $\mu$l) of the pure water (substantially 150 $\mu$l) is transferred (distributed) from the liquid distributor member 60 to the mesh sheet 23 of the extracting cartridge 2 at the dry condition (See Fig. 6).

The result of an experiment for examining the repeatability of pure water transfer (distribution) of the above described method which makes use of the first embodiment will be explained, referring to Fig. 20. In Fig. 20, the horizontal axis represents the amount of pure water carried in the liquid distributor member 60 which is made of cut cotton while the vertical axis presents the distribution ($\mu$l) of pure water transferred from the liquid distributor member 60 to the mesh sheet 23 made of nylon material. In the experiment, the pure water was transferred three times at each case from three different liquid distributor members 60 impregnated with 75 $\mu$l, 150 $\mu$l, and 200 $\mu$l respectively to the mesh sheet 23 through directly abutting each other. The distributed amounts of pure water from the liquid distributor member 60 to the mesh sheet 23 were then measured. The result is shown in Fig. 20 where the average of the distributed amounts from the liquid distributor member 60 to the mesh sheet 23 is about 0.40 $\mu$l when the liquid distributor member 60 carries 75 $\mu$l of the pure water. Also, the average of the distributed amounts from the liquid distributor member 60 to the mesh sheet 23 is about 1.40 $\mu$l when the liquid distributor member 60 carries 150 $\mu$l of the pure water. The average of the distributed amounts from the liquid distributor member 60 to the mesh sheet 23 is about 1.90 $\mu$l when the liquid distributor member 60 carries 200 $\mu$l of the pure water.

It is found that, when the liquid distributor member 60 carries 75 $\mu$l of the pure water, the average of the distributed amounts of pure water from the liquid distributor member 60 to the mesh sheet 23 is found to be substantially 0.5% (0.40 $\mu$l) of the storage (75 $\mu$l) carried in the liquid distributor member 60. When the liquid distributor member 60 carries 150 $\mu$l of the pure water, the average of the distributed amounts of pure water from the liquid distributor member 60 to the mesh sheet 23 is found to be substantially 1.0% (1.40 $\mu$l) of the storage (150 $\mu$l) carried in the liquid distributor member 60. When the liquid distributor member 60 carries 200 $\mu$l of the pure water, the average of the distributed amounts of pure water from the liquid distributor member 60 to the mesh sheet 23 is found to be substantially 1.0% (1.90 $\mu$l) of the storage (200 $\mu$l) carried in the liquid distributor member 60. When the liquid distributor member 60 carries a large amount of pure water (150 $\mu$l and 200 $\mu$), it is found that the transfer of pure water (a ratio of the distributed amount to the storage amount) from the liquid distributor member 60 to the mesh sheet 23 is greater (transfer ration of about 1.0%) when the storage of pure water in the liquid distributor member 60 is abundant (150 $\mu$l to 200 $\mu$l) than when not abundant (75 $\mu$l) as compared with the case in which the liquid distributor member 60 carries a small amount of pure water (transfer ratio of about 0.5%). This may be explained by the fact that the less the storage of pure water, the more the transfer of pure water from the liquid distributor member 60 will be difficult.

It is also found that the transfer of pure water from the liquid distributor member 60 to the mesh sheet 23 exhibits no significant difference between 150 μl and 200 μl of the storage. Accordingly, when the storage of pure water in the liquid distributor member 60 is abundant (150 μl or more) , the transfer of pure water from the liquid distributor member 60 (of absorbent cotton) to the mesh sheet 23 (of nylon material) remains uniform about 1.0 percent. The repeatability of the transfer is substantially 10% as expressed by CV (coefficient of variation: a unit for estimating a variation in data). It is thus proved that the repeatability of the transfer of pure water from the liquid distributor member 60 of cut cotton (absorbent cotton) to the mesh sheet 23 of nylon material is as high as accepted.

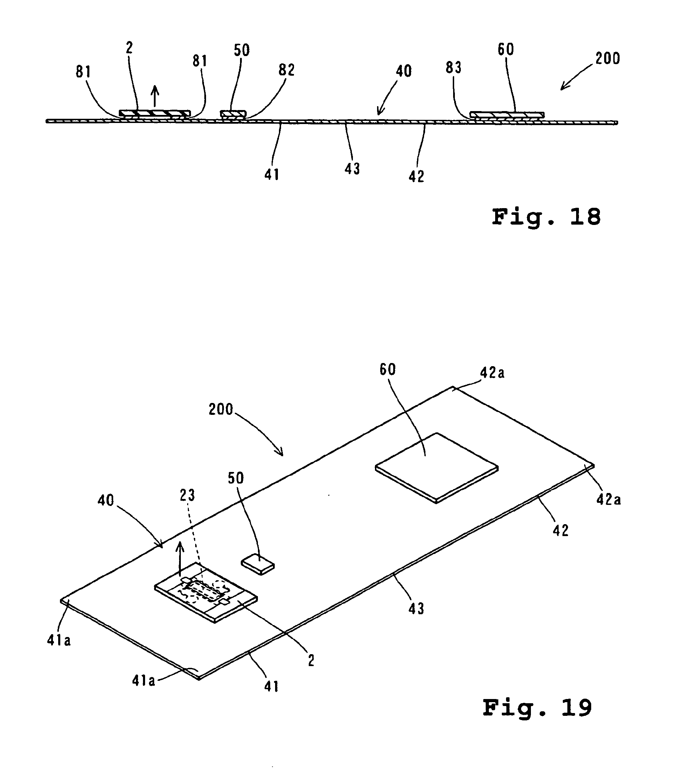

When the transfer of pure water from the liquid distributor member 60 to the mesh sheet 23 has been conducted as described above, both the cartridge support 41 at the two corners 41a and the liquid distributor member support 42 at the two corners 42a of the support member 40 are released from their bonding state. Then, the liquid distributor member support 42 of the support member 40 is turned out in the direction denoted by the arrow D shown in Fig. 17 about the bent 43 used as the fulcrum until it is opened up as shown in Figs. 18 and 19. The extracting cartridge 2 including the mesh sheet 23 impregnated with a predetermined amount (substantially 1.5 μl) of pure water is removed from both the double-coated tapes 81 on the other surface of which the cartridge support 41 is mounted.

This is followed by Step S3 for engaging the two engaging hooks 15 of the blood sugar level measuring apparatus 100 with the corresponding holes 21 of the extracting cartridge 2 thus to load the unused extracting cartridge 2 including the mesh sheet 23 impregnated with a predetermined amount (substantially 1.5 μl) of pure water onto the blood sugar level measuring apparatus 100. At Step S4 for the preparation process, the needle roller 130 (See Fig. 12) is operated to generate rows of tiny extracting apertures 121 in the skin at the wrist of the subject as shown in Fig. 13. This allows the body fluid containing glucose and saved in the dermis to ooze out from the extracting apertures 121 provided by the preparation process with the needle roller 130, as shown in Fig. 21. Step S5 follows where the blood sugar level measuring apparatus 100 is coupled to the fittings of the band member 110. As the result, the blood sugar level measuring apparatus 100 is mounted to the wrist 120 of the subject with its extracting cartridge 2 remaining at the lower surface in direct contact with the skin. More particularly as shown in Fig. 5, with the band member 110 (See Fig. 1) tightened up thus to raise the measuring (extracting) portion of the wrist 120, the mesh sheet 23 comes into direct contact with the skin and is lifted at the center, hence becoming in contact with the lower surface (the measuring surface 26a) of the sensor member 26.

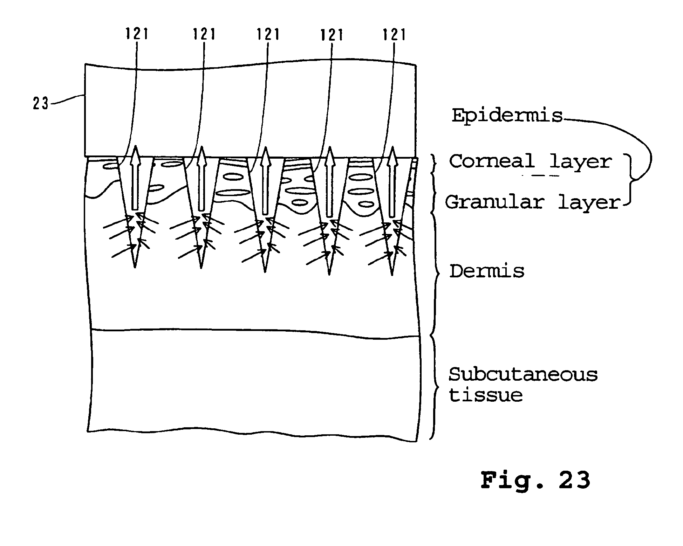

Simultaneously, the pure water carried in the mesh sheet 23 moves into the extracting apertures 121 in the skin. Upon mixing up with the pure water from the mesh sheet 23, the body fluid oozing out from the extracting apertures 121 in the skin is dispersed into the pure water carried in the mesh sheet 23, as shown in Fig. 23. This causes the osmotic pressure in the extracting apertures 121 to be lower than the osmotic pressure in the dermis, thus encouraging the transfer of the body fluid from the dermis to the extracting apertures 121. Accordingly, the body fluid oozing out from the extracting apertures 121 remains dispersed in the pure water carried in the mesh sheet 23 before the anode 24 and the cathode 25 in the extracting cartridge 2 are loaded with a constant voltage from the constant voltage source 13. Then, when the start switch (not shown) is turned on at Step S6 shown in Fig. 14 for starting the action of the blood sugar level measuring apparatus 100, the constant voltage source 13 applies a constant voltage (of 0.8 V) to between the anode 24 and the cathode 25 for about three minutes. This allows charged ions in the extracting apertures 121 to actively immigrate towards the anode 24 and the cathode 25. As the result, the body fluid containing a detectable amount of glucose to be measured by the detector 3 can be captured in the pure water carried in the mesh sheet 23, as shown in Fig. 23.

The mechanism of the body fluid containing a detectable amount of glucose being captured in the pure water carried in the mesh sheet 23 by the effect of application of the voltage between the anode 24 and the cathode 25 in the extracting cartridge 2 will now be described. When the anode 24 and the cathode 25 are loaded with the constant voltage from the constant voltage source 13, their collector electrodes 24a and 25a receive a positive (+) charge and a negative (-) charge respectively. The active carbon electrodes 24b and 25b provided beneath the two collector electrodes 24a and 25a of the anode 24 and the cathode 25 respectively have polarizability. Accordingly, the active carbon electrode 24b at the anode 24 is charged with positive (+) electricity while the active carbon electrode 25b at the cathode 25 is charged with negative (+) electricity. This causes sodium ions ($Na^+$) and chloride ions ($Cl^-$) in the body fluid to escape from the extracting apertures 121, pass through the pure water carried in the mesh sheet 23, and immigrate towards the active carbon electrodes 25b and 24b respectively. As the sodium ions ($Na^+$) and the chloride ions ($Cl^-$) in the body fluid are immigrated towards the active carbon electrodes 25b and 24b respectively, the biochemical components including glucose are transferred and captured in the pure water carried in the mesh sheet 23. The biochemical components including glucose in the body fluid are finally received by the measuring surface 26a of the sensor member 26.

As the action at Step S6 being conducted, the glucose level is measured at Step S7 using the sensor member 26. The measurement of glucose level is repeatedly carried out at equal intervals of time (for example, at every one second) with the anode 24 and the cathode 25 being loaded with the constant voltage from the constant voltage source 13.

More specifically, by the catalytic action of glucose oxidase (GOD), which is a reducing enzyme in the gel mixture applied on the measuring surface 26a of the sensor member 26, the glucose received by the measuring surface 26a is converted into hydrogen peroxide ($H_2O_2$) and gluconic acid. Then, by the catalytic action of per-oxydase (POD), which is an oxidizing enzyme in the gel mixture applied on the measuring surface 26a of the sensor member 26, hydrogen peroxide ($H_2O_2$) is converted into active oxygen ($O^*$) and water ($H_2O$). This allows the gel mixture on the measuring surface 26a of the sensor member 26 to react with the active oxygen ($O^*$), thus producing a color.

Accordingly, the light which is fully reflected on and passed through the second optical waveguide layer 26d (See Fig. 7) of the sensor member 26 with which the mesh sheet 23 impregnated with pure water comes into direct contact is received by the light receiving element 34 after absorbed in the coloring agent which produces a color in response to the extracted amount of glucose. A signal output of the light receiving element 34 is transferred to the controller 11 where it is used for calculating the amount of glucose. The coloring agent contained in the pure water may be N,N-bis(2-hydroxy-3-sulphoporpyl)-tolidine-di-potassic-sal t or 3,3',5,5'-tetramethyl-benzilidene. Step S8 then follows where the blood sugar level is calculated from the amount of glucose determined every one second at Step S7 by the controller 11 using the following equation (1) and then displayed together with the extracted amount per unit time of glucose on the display 12 (See Fig. 1).

$$BG = C/P = C/(A \times I + B) \qquad ...(1)$$

where "BG" is the calculated blood sugar level, C is the extracted amount per unit time of glucose calculated every one second at Step S7, P is the transmissivity of glucose across the extracting area (the level of ease for passing glucose), I is the average of currents measured with a current meter 14 during the voltage application, and A and B are the constants predetermined through a series of preparatory experiments. At Step S9 shown in Fig. 14, the blood sugar level measuring apparatus 100 is removed from the fittings of the band member 110.

Step S10 follows this for disengaging the engaging holes 21 of the extracting cartridge 2 from the hooks 15 of the blood sugar level measuring apparatus 100 to separate the used extracting cartridge 2 from the blood sugar level measuring apparatus 100. The used extracting cartridge 2 maybe discarded or subjected to a recovery process for reuse. Then at Step S11, the band member 110 is dismounted from the wrist 120 of the subject. The procedure of the blood sugar level measuring apparatus 100 measuring the blood sugar level is now completed.

According to the first embodiment, the liquid distributor member 60 is made of absorbent cotton (cut cotton) for carrying a given amount (substantially 150 $\mu$l) of pure water and arranged to come into direct contact with the mesh sheet 23, made of dried nylon material, of the extracting cartridge 2 for distributing the pure water to the mesh sheet 23. This allows the pure water to be transferred from the liquid distributor member 60 to the mesh sheet 23 regardless of the condition of the extracting cartridge 2 (whether or not the mesh sheet 23 is impregnated with predetermined amount of pure water prior to the use of the cartridge), hence increasing the storage period of the extracting cartridge 2. Also, the mesh sheet 23 can favorably be impregnated with pure water by a simple action of transferring the pure water from the liquid distributor member 60 to the mesh sheet 23.

According to the first embodiment, the liquid distributor member 60 is made of absorbent cotton (cut cotton) for carrying a given amount (substantially 150 $\mu$l) of pure water and arranged to come into direct contact with the mesh sheet 23, made of dried nylon material, of the extracting cartridge 2 for distributing a uniform amount of the pure water to the mesh sheet 23. By this contact, the liquid distributor member 60 made of absorbent cotton (cut cotton) distributes a substantially constant amount of pure water. As the result, the mesh sheet 23 can controllably be supplied with as a trace (amount as 1.5 $\mu$l in the first embodiment) of the pure water.

According to the first embodiment, the separator member 70 is provided for separating but not contacting between the mesh sheet 23 of the extracting cartridge 2 and the liquid distributor member 60 while the extracting cartridge 2 and the liquid distributor member 60 are accommodated in the support member 40. This allows the extracting cartridge 2 to be securely held in its stored condition as physically separated by the separator member 70 from the liquid distributor member 60.

According to the first embodiment, the extracting cartridge set 200 is arranged where the separator member 70 is removed from the support member 40 to allow the direct contact between the liquid distributor member 60 and the mesh sheet 23 of the extracting cartridge 2. As the result, the mesh sheet 23 can be supplied with pure water by such a simple action as described.

According to the first embodiment, the mesh sheet 23 of the extracting cartridge 2 is supplied with pure water through directly contacting with the liquid distributor member 60. This can prevent the pure water from being fouled by the direct contact with fingers or external components, hence ensuring the precise action of analyzing the glucose.

According to the first embodiment, the mesh sheet 23 remains in its dry condition while being stored in the extracting cartridge 2. This canprevent the enzymes (glucose oxidase (GOD) and per-oxydase (POD)) in the sensor member 26 from being deteriorated within a short period of time when coming into direct contact with water.

According to the first embodiment, the separator member 70 is arranged for physically separating the mesh sheet 23 of the extracting cartridge 2 from the liquid distributor member 60. Since the mesh sheet 23 of the extracting cartridge 2 is kept with no contact with the liquid distributor member 60 by the function of the separator member 70, the mesh sheet 23 can be easily stored in its dry condition in the support member 40.

According to the first embodiment, the mesh sheet 23 of the extracting cartridge 2 and the liquid distributor member 60 are disposed on both surfaces of the separator member 70 so as to be opposed to each other in the support member. This allows the mesh sheet 23 of the extracting cartridge 2 to easily come into direct contact with the liquid distributor member 60 when the separator member 70 is removed out from between the mesh sheet 23 and the liquid distributor member 60 by accommodating both the extracting cartridge 2 and the liquid distributor member 60 in the support member 40.

Additionally, according to the first embodiment, the support member 40 is made of a flexible material. This allows the direct contact between the mesh sheet 23 and the liquid distributor member 60 to be conducted by pressing and deforming with the fingers from both surfaces the particular location of the support member 40 where the mesh sheet 23 and the liquid distributor member 60 are accommodated opposite to each other when having removed out the separator member 70 from the support member 40, thus readily supplying the mesh sheet 23 with pure water from the liquid distributor member 60.

According to the first embodiment, the insertion 73 of the separator member 70 is detachably held between the cartridge holder 71 for holding the extracting cartridge 2 and the liquid distributor member holder 72 for holding the liquid distributor member 60 in the support member 40. This allows both the cartridge holder 71 and the liquid distributor member holder 72 to be removed out from the support member 40 at one time when the insertion 73 is pulled out by the subject and its pulled force is transmitted to the cartridge holder 71 and the liquid distributor member holder 72. As the result, the extracting cartridge 2 can come into direct contact with the liquid distributor member 60.

According to the first embodiment, the mesh sheet 23 has a porous construction, which can thus be impregnated at its multiple pores with pure water supplied from the liquid distributor member 60. Also, since the mesh sheet 23 is made of a nylon material, its change in the thickness upon being pressed down against the skin of the subject can be minimized, thus maintaining the distance between the skin and the sensor member 26 uniform with ease.

According to the first embodiment, the extracting cartridge 2 has the anode 24 and the cathode 25 connected to the positive port and the negative port respectively of the constant voltage source 13 in the blood sugar level measuring apparatus 100. This allows two flows of glucose moving towards the anode 24 and the cathode 25 respectively to be captured at once by the extracting cartridge 2. As the result, the action of the subject can be simplified as compared with a prior art arrangement where the two flows of glucose moving towards the anode 24 and the cathode 25 are captured by two separate extracting cartridges corresponding to the anode 24 and the cathode 25 respectively.

(Second Embodiment)

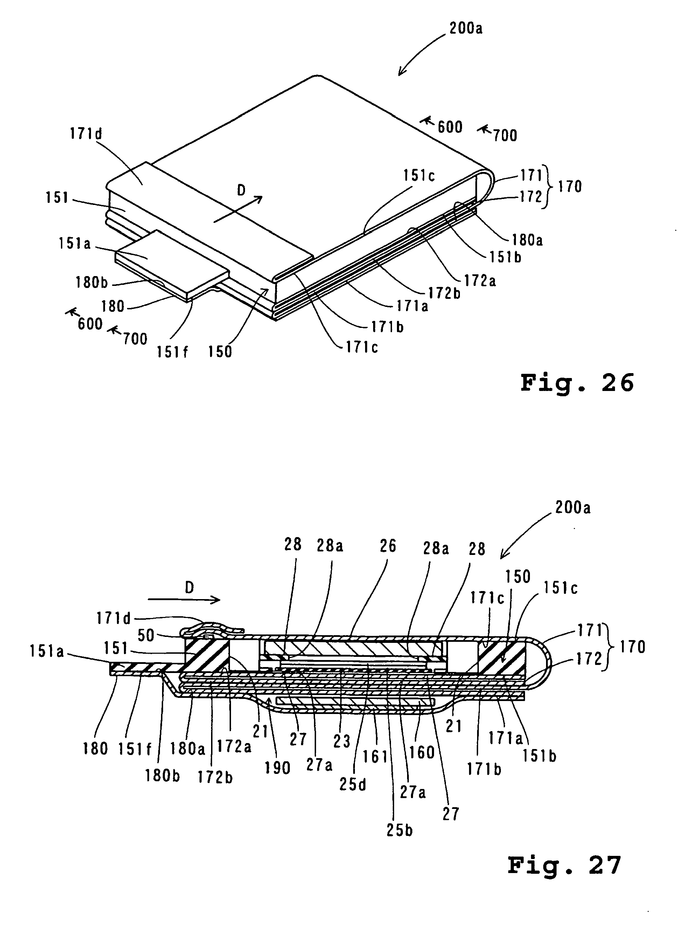

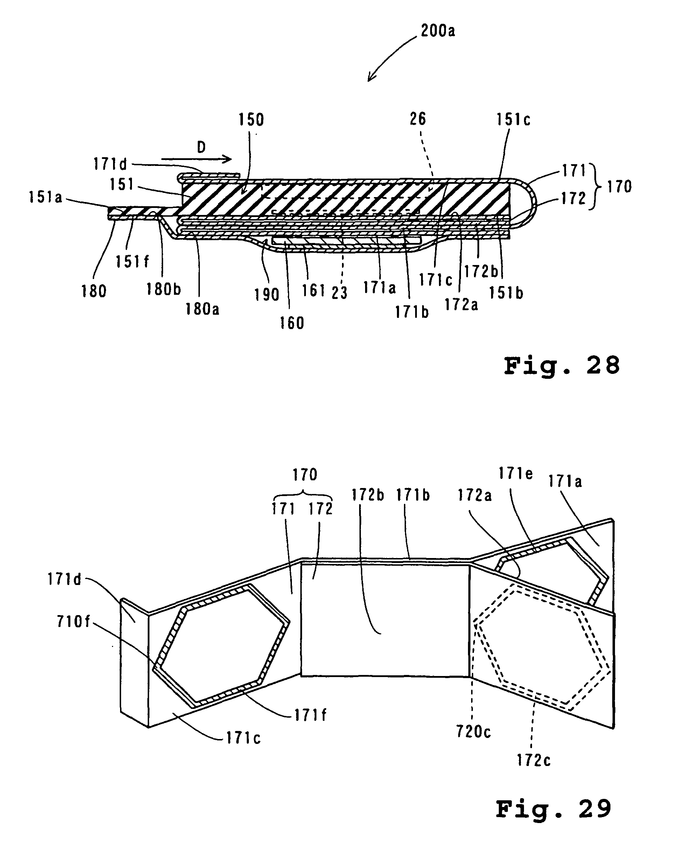

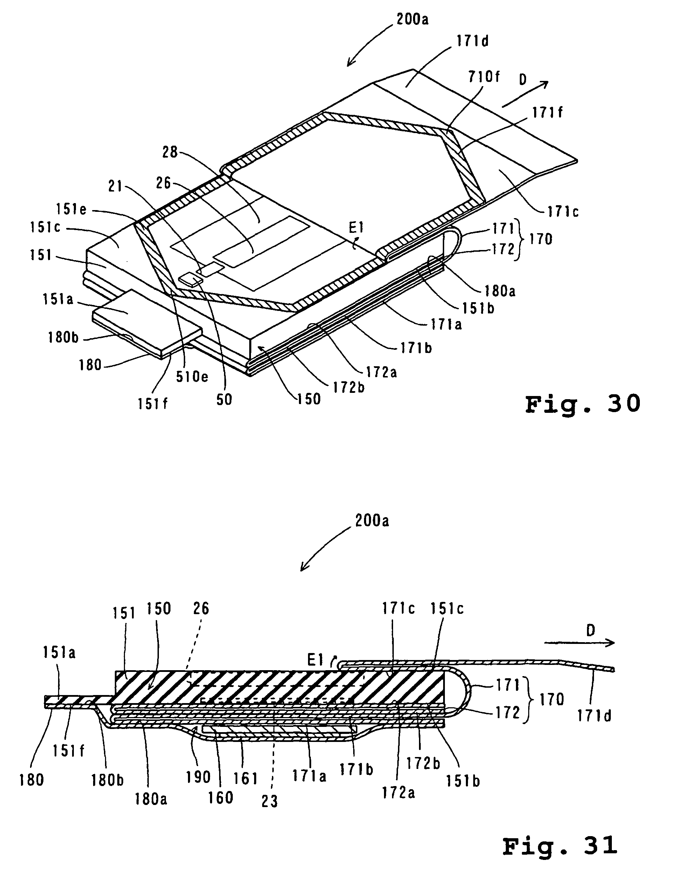

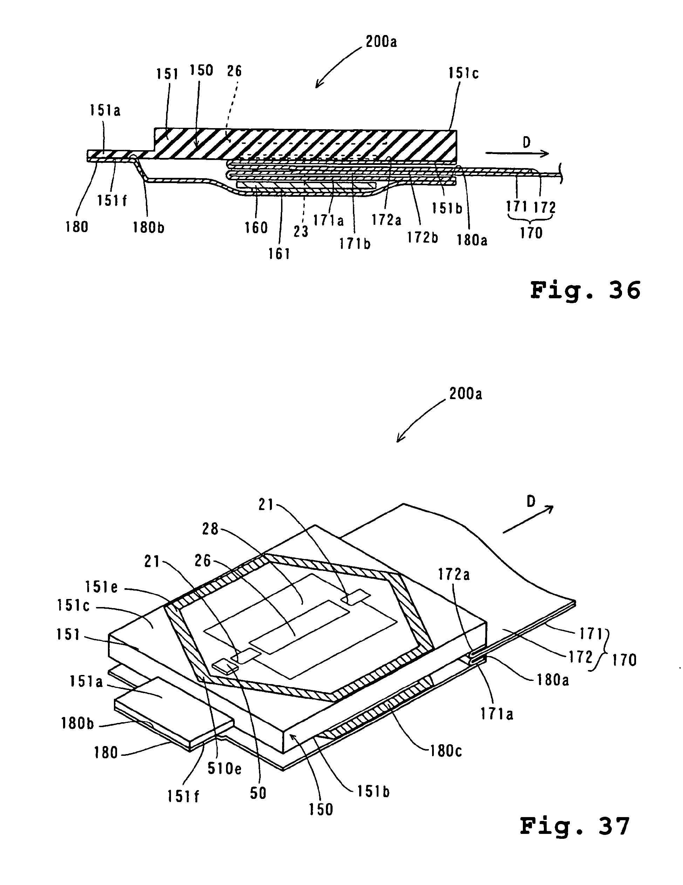

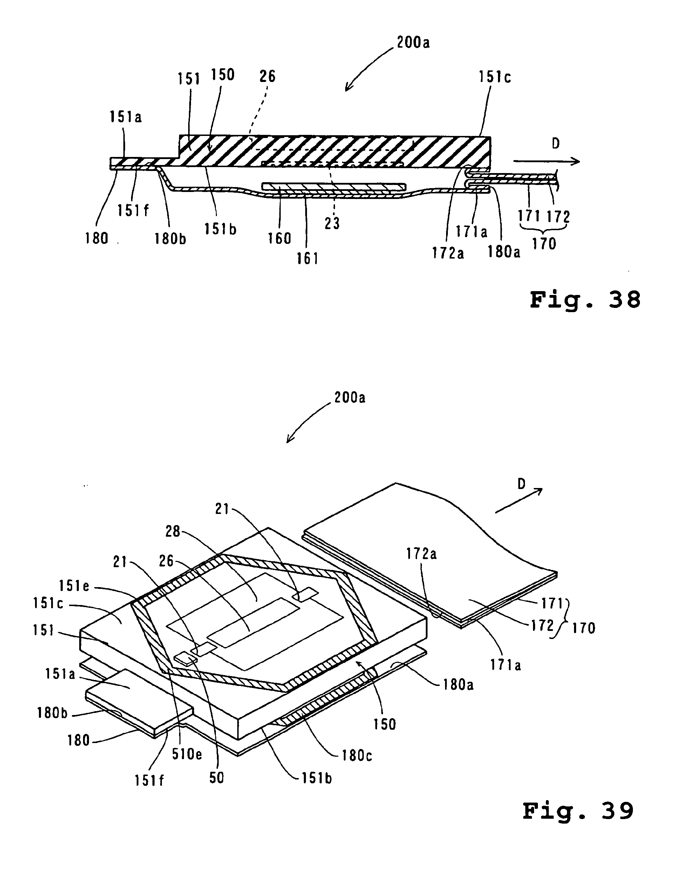

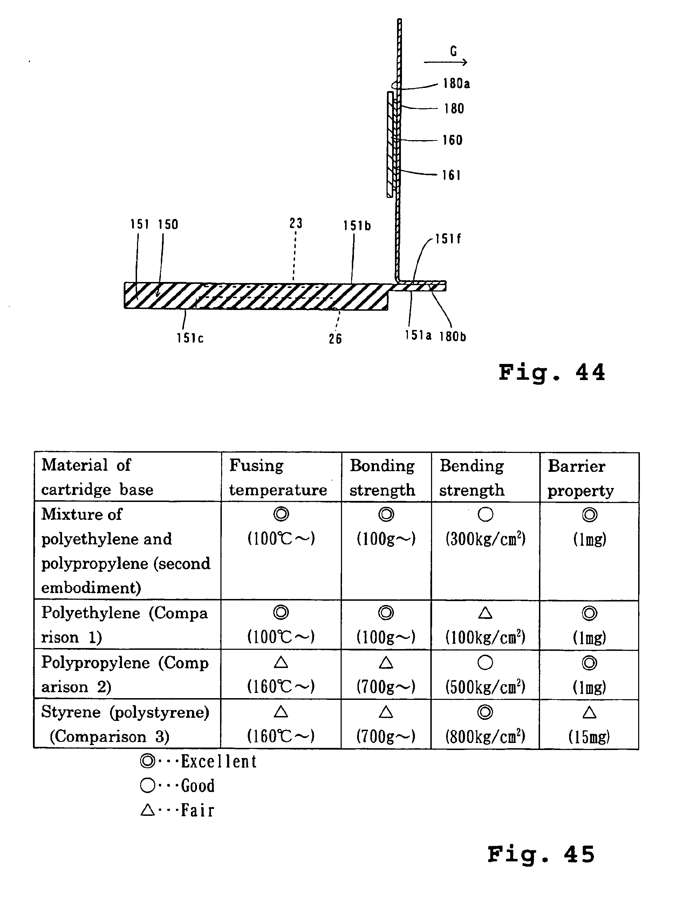

Figs. 26 to 28 illustrate an extracting cartridge set according to the second embodiment of the present invention. Fig. 29 is a perspective view of a separator member in the extracting cartridge set of the second embodiment. Figs. 30 to 44 are explanatory views showing a construction and its action of the extracting cartridge set of the second embodiment. Fig. 45 is a profile showing the fusing characteristics of an IMX film used as the material of a cartridge base according to the second embodiment. Referring to Figs. 26 to 45, the extracting cartridge set 200a of the second embodiment will be described in more detail, which is different in the construction from that of the first embodiment. The arrangements other than the extracting cartridge set 200a in the second embodiment are substantially identical to those of the first embodiment.

The extracting cartridge set 200a of the second embodiment is arranged substantially equal to the extracting cartridge set 200 of the first embodiment, where the mesh sheet 23 (See Fig. 27) in an unused cartridge 150 to be loaded onto the blood sugar level measuring apparatus 100 is held together with the sensor member 26 in the dry condition and can be supplied with a given amount of pure water when loaded to the blood sugar level measuring apparatus 100. The extracting cartridge set 200a of the second embodiment comprises, as shown in Fig. 27, a desiccator 50, the extracting cartridge 150, a liquid distributor member 160 impregnated with pure water, a separator member 170, and a housing member 180.

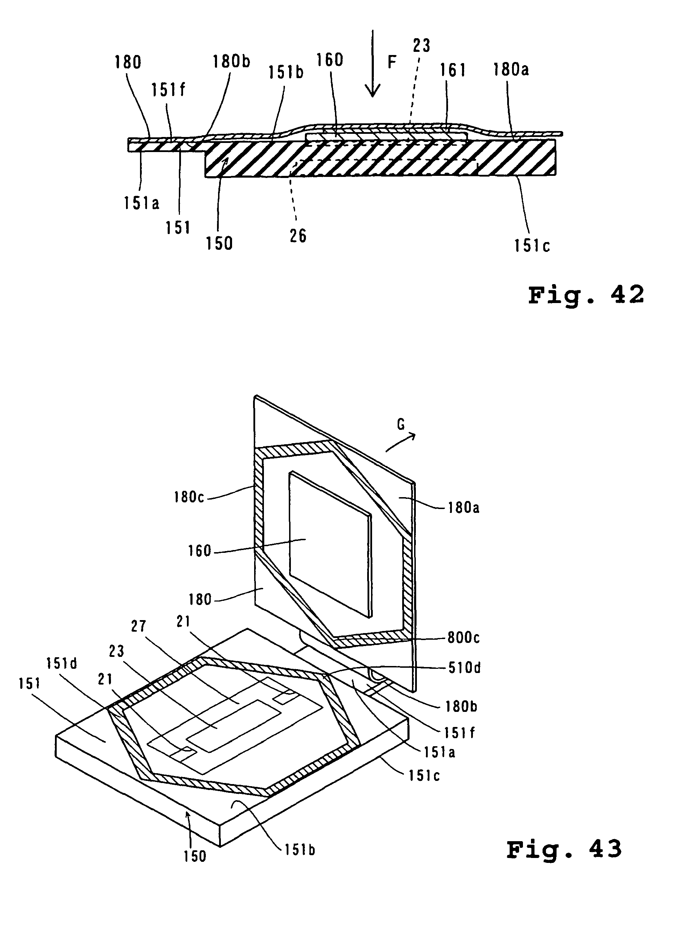

The extracting cartridge 150 is substantially similar in the arrangement to the extracting cartridge 2 of the first embodiment shown in Fig. 6 and particularly includes a grip 151a. More specifically, the extracting cartridge 150 includes, as shown in Fig. 27, a cartridge base 151, a mounting opening 21, a mesh sheet 23, an active carbon electrode 25b, an operator 25d, a sensor member 26, a double-faced tape 27, and an electrode sheet 28. The cartridge base 151 is arranged integral with the grip 151a which extends in one direction and can be gripped by the fingers of a subject to be examined for taking out the separator 170 from the extracting cartridge 150 . The grip 151a is smaller in thickness than the cartridge base 151. The housing member 180 is joined by thermally fusing to the grip 151a of the cartridge base 151. The cartridge base 151 has a holding area 151d (See Fig. 43) of a hexagonal shape in the plan view provided on one surface 151b thereof for holding the separator member 170 and another holding area 151e (See Fig. 30) of a hexagonal shape in the plan view provided on the other surface 151c thereof for holding the separator member 170.

The desiccator 50 is detachably mounted to the other surface 151c of the cartridge base 151 as spaced by a distance from the sensor member 26.

In the second embodiment, the cartridge base 151 is made of a resin material including PE (polyethylene) and PP (polypropylene). The ratio (by weight) of PE (polyethylene) and PP (polypropylene) contained in the cartridge base 151 may preferably be within a range from 1: 2 to 2 : 1 or more preferably PE:PP=1:1.

The separator member 170 and the housing member 180 in the second embodiment are made of a flexible sheet material. More particularly, the separator member 170 and the housing member 180 are made of IMX film, a product of J-Film Corporation. Each of the separator member 170 and the housing member 180 has a three-layer construction including a PET (polyethylene terephthalate) layer, an aluminum foil, and a PE (polyethylene) layer laminated in this order. The PE layer serves as an adhesive (fusing) layer. Bonded between the PET layer and the aluminum foil and between the aluminum foil and the PE layer are by adhesive layers of a PE (polyethylene) material.

The separator member 170 in the second embodiment is a combination of two bonding members 171 and 172 joined together as shown in Fig. 29. The bonding member 171 comprises a bonding surface 171a, a separator portion 171b, another bonding surface 171c, and a grip portion 171d. The bonding surfaces 171a and 171c have bonding portions 171e and 171f of a hexagonal shape in the plan view respectively. The bonding surface 171a of the bonding member 171 is bonded at its bonding portion 171e of the hexagonal shape with the housing member 180 (See Fig. 28), thus forming a liquid distributor member housing portion 190. The bonding surface 171c of the bonding member 171 is bonded at its bonding portion 171f of the hexagonal shape with the bonding area 151e on the other surface 151c of the cartridge base 151 (See Fig. 30), as shown in Fig. 29. The grip 171d of the bonding member 171 is provided for being gripped by the subject for taking out the separator member 170 from the extracting cartridge 150.

Also as shown in Fig. 29, the bonding member 172 comprises a bonding surface 172a and a separator surface 172b. The bonding surface 172a has a bonding portion 172c of a hexagonal shape in the plan view. The bonding surface 172a of the bonding member 172 is bonded at its bonding portion 172c of the hexagonal shape with the bonding area 151d on the one surface 151b of the cartridge base 151 (See Fig. 43). The bonding potion 171f of the bonding surface 171c and the bonding surface 172a at the bonding portion 172c of the separator member 170 are bonded to the bonding area 151e on the other surface 151 and the bonding area 151d on the one surface 151b of the cartridge base 151 respectively, whereby both the surfaces 151b and 151c of the cartridge base 151 are sealed off by the two bonding surfaces 172a and 171c of the separator member 170. This allows the mesh sheet 23 of the extracting cartridge 150 to be accommodated (held) in the sealed space between the separator member 170 and the cartridge base 151.

The bonding between the bonding potion 171f of the bonding surface 171c of the separator member 170 and the bonding area 151e on the other surface 151c of the cartridge base 151 and the bonding between the bonding surface 172a at the bonding portion 172c of the separator member 170 and the bonding area 151d on the one surface 151b of the cartridge base 151 are implemented by thermally fusing and bonding the PE (polyethylene) layers of the bonding surface 171c at the bonding portion 171f and the bonding surface 172a at the bonding portion 172c to the bonding area 151e of the other surface 151c and the bonding area 151d of the one surface 151b of the cartridge base 151 respectively. The thermal fusing may involve heating at substantially 100°C to 180°C (for example, 140 °C) for substantially 0.1 second to 10 seconds (for example, 4 seconds).

The separator surfaces 171b and 172b shown in Fig. 29 are joined together by thermally fusing and bonding the PE (polyethylene) layer at the outside of the separator portion 171b to the PE (polyethylene) layer at the outside of the separator surface 172b. The bonding strength between the two separator surfaces 171b and 172b is much greater than those between the bonding surface 171c of the separator member 170 and the other surface 151c of the cartridge base 151, between the bonding surface 172a of the separator member 170 and the one surface 151b of the cartridge base 151, and between the bonding surface 171a of the separator member 170 and the housing member 180. For increasing the bonding strength between the two separator surfaces 171b and 172b, the action of heating at substantially 100 °C to 180 °C (for example, 140 °C) for substantially 0.1 second to 10 seconds (for example, 4 seconds) may be repeated several times (for example, two times).

The housing member 180 in the second embodiment comprises, as shown in Figs. 28 and 43, a housing member bonding surface 180a joined with the bonding surface 171a of the bonding member 171 (See Fig. 28) and a housing member mounting portion 180b joined with the one surface 151f of the grip 151a of the cartridge base 151. The housing member bonding surface 180a has a housing member bonding portion 180c of a hexagonal shape in the plan view. The liquid distributor member 160 is securely bonded by an adhesive 161 to the housing member bonding surface 180a of the housing member 180, as shown in Fig. 44.

The bonding between the bonding surface 171a of the bonding member 171 of the separator member 170 and the housing member bonding surface 180a of the housing member 180 is implemented by thermally fusing and bonding the PE (polyethylene) layer at the outside of the bonding surface 171a to the PE (polyethylene) layer at the outside of the housing member bonding portion 180c of the housing member bonding surface 180a. The thermal fusing may involve heating at substantially 100°C to 180°C (for example, 140°C) for substantially 0.1 second to 10 seconds (for example, substantially 4 seconds).

Similarly, the bonding between the housing member mounting portion 180b of the housing member 180 and the grip 151a of the cartridge base 151 is implemented by thermally fusing and bonding the PE (polyethylene) layer at the outside of the housing member mounting portion 180b to the PE (polyethylene) layer at the outside of the one surface 151f of the grip 151a of the cartridge base 151. The thermal fusing may involve heating the entire surface of the one surface 151f at substantially 100 °C to 180°C (for example, 140°C) for substantially 0.1 second to 10 seconds (for example, 4 seconds), as shown in Figs. 43 and 44.

The liquid distributor member housing portion 190 is provided as an enclosed space of a hexagonal shape in the plan view defined by bonding between the bonding portion 171e of the bonding surface 171a of the separator member 170 and the housing member bonding portion 180c of the housing member bonding surface 180a of the housingmember 180. As the result, the liquid distributor member 160 remains airtightly accommodated (held) in the liquid distributor member housing portion 190.

The action of taking out the separator member 170 from the extracting cartridge set 200a will now be explained referring to Fig. 26, Figs. 28 to 40, and Fig. 43.

As shown in Figs. 26 and 28, the grip 151a of the cartridge base 151 is gripped together with the grip portion 171d of the separator member 170 in the extracting cartridge set 200a and pulled in the (taking out) direction denoted by the arrow D. This causes the bonding surface 171c of the separator member 170 to be urged with a counter stress in the (peeling) direction E1 at the other end opposite to the (taking out) direction D and peeled off from the other end, as shown in Figs. 30 and 31, it starts separating from the other end opposite to the (taking out) direction D of the bonding portion 171c. Since the bonding surface 171c of the separator member 170 is bonded at the bonding portion 171f of the hexagonal shape to the bonding area 151e on the other surface 151c of the cartridge base 151, its peeling action starts from the corner 710f of the bonding portion 171f at the corner 510e of the bonding area 151e to separate the bonding surface 171c at the bonding portion 171f from the bonding area 151e of the other surface 151c. As the result, the pulling force required for separating the bonding surface 171c from the other surface 151c can be decreased as compared with the bonding area of a square shape.

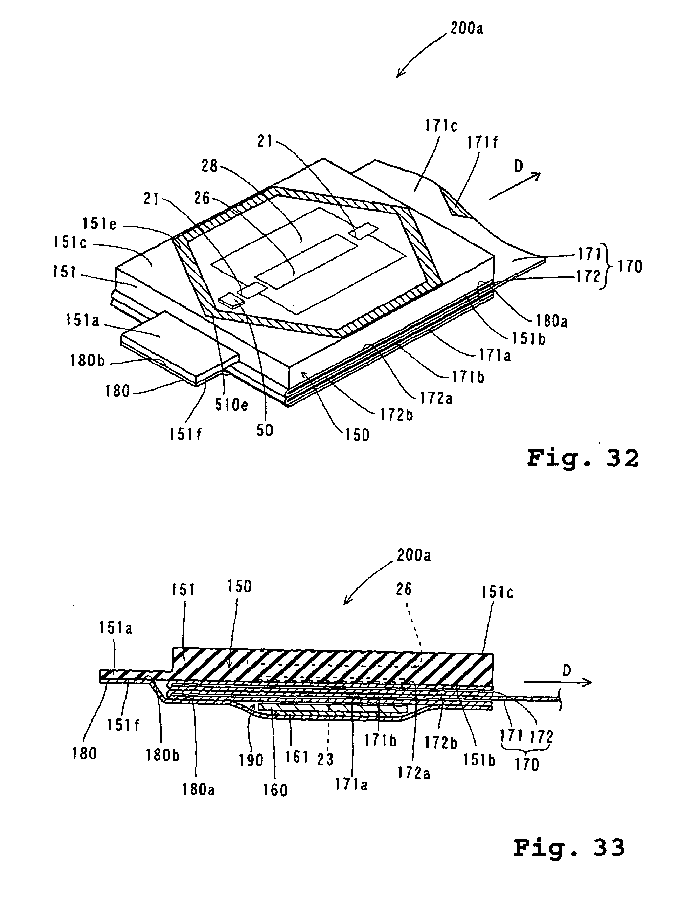

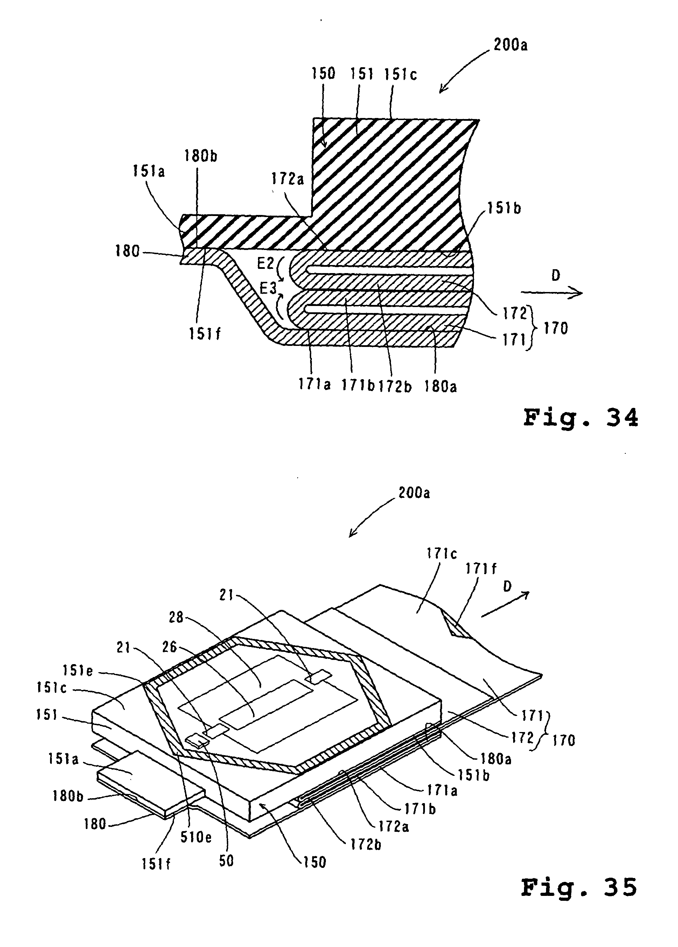

As shown in Figs. 32 and 33, the grip 151a of the cartridge base 151 remains gripped together with the grip portion 171d of the separator member 170 (See Fig. 26) and continuously pulled in the (taking out) direction denoted by the arrow D when the bonding surface 171c of the separator member 170 has been separated from the other surface 151c of the cartridge base 151. The pulling force applied to the grip portion 171d of the bonding member 171 of the separator member 170 in the (taking out) direction D is transmitted to not only the bonding member 171 but also the bonding member 172 because the two bonding members 171 and 172 are joined together between their respective separator surfaces 171b and 172b. When the bonding surface 172a of the bonding member 172 of the separator member 170 is urged with the pulling force in the (peeling) direction E2 at the other end opposite to the (taking out) direction D as show in Fig. 34, it starts separating from the other end opposite to the (taking out) direction D of the bonding surface 172a. Equally, when the bonding surface 171a of the bonding member 171 of the separator member 170 is urged with the pulling force in the (peeling) direction E3 at the other end opposite to the (taking out) direction D, it starts separating from the other end opposite to the (taking out) direction D of 171a.

Also, since the bonding surface 172a of the separator member 170 is bonded at the bonding portion 172c of the hexagonal shape (See Fig. 29) to the bonding area 151d on the one surface 151b of the cartridge base 151 (See Fig. 43), its peeling action starts from the corner 720c of the bonding portion 172c (See Fig. 29) at the corner 510d of the bonding area 151d (See Fig. 43) to separate the bonding surface 172a at the bonding area 171c from the bonding area 151d of the one surface 151b. As the result, the pulling force required for separating the bonding surface 172a from the one surface 151b can be decreased as compared with the bonding area of a square shape. Similarly, since the bonding surface 171a of the separator member 170 is bonded at the bonding portion 171e of the hexagonal shape (See Fig. 29) to the housing member bonding portion 180c on the housing member bonding surface 180a of the housing member 180 (See Fig. 43), its peeling action starts from the corner (not shown) of the bonding portion 171e at the corner 800c of the housing member bondingportion 180c (See Fig. 43) to separate the bonding surface 171a at the bonding portion 171e from the housing member bonding portion 180c of the housing member bonding surface 180a. As the result, the pulling force required for separating the bonding surface 171a from the housing member bonding surface 180a can be decreased as compared with the bonding area of a square shape.

As shown in Figs. 35 to 40, the separator member 170 can be separated from the extracting cartridge 150 with its two bonding surfaces 172a and 171a removing from the one surface 151b of the cartridge base 151 and the housing member bonding surface 180a of the housing member 180 respectively. Through the foregoing steps, the separator member 170 is taken out from the extracting cartridge set 200a.

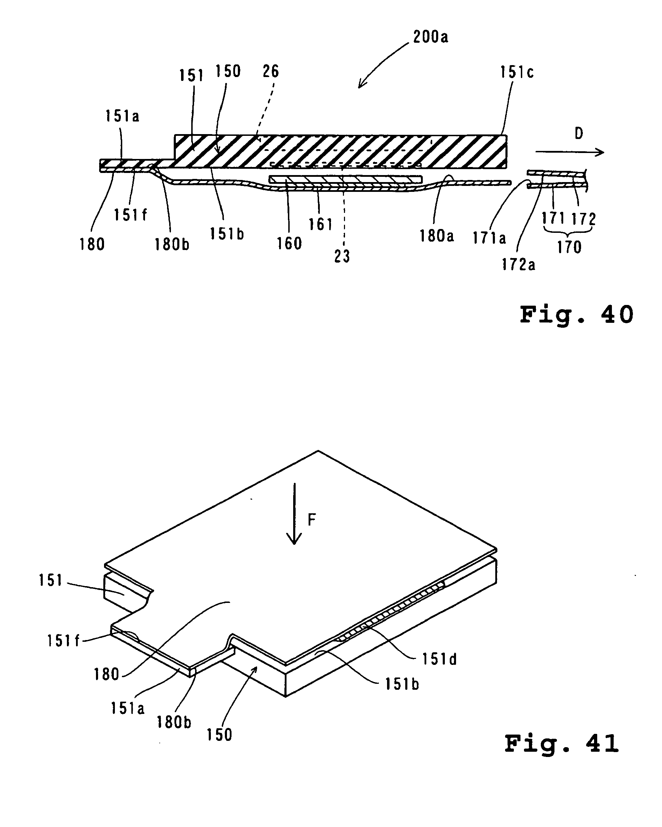

When the separator member 170 has been taken out from the extracting cartridge set 200a, the action follows for pressing down the surface of the housingmember 180 opposite to the surface where the liquid distributor member 160 is mounted thus to depress the liquid distributor member 160 impregnated with pure water in the direction denoted by the arrow F as shown in Figs. 41 and 42. This causes the liquid distributor member 160 impregnated with pure water to come into direct contact with the mesh sheet 23 in the dry condition (See Fig. 43) in the extracting cartridge 150 and distribute (feed) its pure water to the mesh sheet 23 in the extracting cartridge 150. Then, when the housing member 180 is pulled in the direction denoted by the arrow G shown in Fig. 43, it can be separated from the extracting cartridge 150.

According to the second embodiment, as described above, the separator member 170 is detachably joined to the bonding member 180 and extracting cartridge 150 separated therefrom, for permitting the contact between the mesh sheet 23 and the liquid distributor member 160. This allows the contact between the mesh sheet 23 and the liquid distributor member 160 to be conducted simply separating from the housing member 180 by the separator 170 and the extracting cartridge 150. As the result, the mesh sheet 23 can readily be supplied with pure water from the liquid distributor member 160.

According to the second embodiment, the housing member 180 has the housing member bonding surface 180a while the separator member 170 has the bonding surface 171a bonded to the housing member bonding surface 180a of the housing member 180, the bonding surface 172a bonded to the cartridge base 151 of the extracting cartridge 150, and the bonding surface 171c and 172 a are provided, so that the enclosed space for accommodating the liquid distributor member 160 is defined between the housing member bonding surface 180a of the housing member 180 and the bonding surface 171a of the separator member 170 and the other enclosed space is defined by the two bonding surfaces 172a and 171c of the separator member 170 and the cartridge base 151 of the extracting cartridge 150 which includes the mesh sheet 23. This allows the liquid distributor member 160 and the mesh sheet 23 of the extracting cartridge 150 to be isolated and stored in their respective enclosed spaces.

According to the second embodiment, the liquid distributor member 160 is mounted to the housing member bonding surface 180a of the housing member 180 which is in turn arranged to remain bonded at the housing member mounting portion 180b to the extracting cartridge 150 after removal of the separator member 170. This allows the liquid distributor member 160 to remain not removed from the housing member 180 when the separator member 170 has been separated from the extracting cartridge 150, hence facilitating the handling action. Also, since the housing member mounting portion 180b of the housing member 180 remains joined to the grip portion 151a of the extracting cartridge 150 with the liquid distributor member 160 added to the location opposite to the mesh sheet 23 of the housing member bonding surface 180a of the housing member 180, the mesh sheet 23 in the extracting cartridge 150 can certainly face the liquid distributor member 160 after removal of the separator member 170. As the result, the pure water can readily be distributed from the liquid distributor member 160 to the mesh sheet 23 by pressing down the housing member 180 at the surface opposite to the housing member bonding surface 180a in the direction F with fingers (See Fig. 42) after removal of the separator member 170. Moreover, the liquid distributor member 160 is mounted to the housing member bonding surface 180a side of the housing member 180, allowing its impregnated pure water to transfer or distribute to the mesh sheet 23 by pressing down the housing member 180 at the surface opposite to the housing member bonding surface 180a in the direction F (See Fig. 42). As the result, the liquid distributor member 160 on the housing member bonding surface 180a and its distributing water to the mesh sheet 23 are prevented from coming into direct contact with the fingers of the subject and thus can be protected from being fouled.

According to the second embodiment, the housing member 180 has the housing member bonding surface 180a on which the liquid distributor member 160 is mounted and the housing member mounting portion 180b detachably bonded to the cartridge base 151 of the extracting cartridge 150, whereby the housing member 180 is detachably joined by its housing member mounting portion 180b to the cartridge base 151 of the extracting cartridge 150. This allows the housing member 180 to be separated together with the liquid distributor member 160 from the cartridge base 151 of the extracting cartridge 150 when the mesh sheet 23 in the extracting cartridge 150 has been supplied with pure water from the liquid distributor member 160.

According to the second embodiment, the extracting cartridge 150 has the grip 151a thereof arranged to be gripped for separating the separator member 170 from the extracting cartridge 150. This allows the separator member 170 to be readily separated from the extracting cartridge 150 with the grip 151a of the extracting cartridge 150 being held.

According to the second embodiment, the separator member 170 has the bonding portion 171e detachably bonded to the housing member bonding surface 180a of the housing member 180, the separator surfaces 171b and 172b for isolating the mesh sheet 23 in the extracting cartridge 150 form the liquid distributor member 160, the bonding portion 172c detachably bonded to the one surface 151b of the cartridge base 151, and the bonding surface 171f detachably bonded to the other surface 151c of the cartridge base 151. This allows the liquid distributor member 160 on the housing member 180 and the mesh sheet 23 in the extracting cartridge 150 to be held in their respective enclosed spaces defined by the bonding portions 171e, 172c, and 171f while they are isolated from each other by the separator portions 171b and 172b.

According to the second embodiment, the bonding portions 171e, 172c, and 171f of the separator member 170 are arranged to peel and separate along the corresponding directions of peel-off (denoted by E3, E2, and E1 respectively) from the end opposite to the direction (denoted by the arrow D) of removing the separator member 170 by a pulling force applied in the direction D to the separator member 170, and this it starts separating from the other end opposite to the (taking out) direction D of the separator member 170. This allows the bonding portions 171e, 172c, and 171f of the separator member 170 to be readily separated from the extracting cartridge 150 by urging the separator member 170 with a pulling force in the direction of separation (denoted by the arrow D).

According to the second embodiment, the separator member 170 has the grip portion 171d arranged to be gripped for separating the separator member 170 at least from the extracting cartridge 150. This allows the separator member 170 to be easily urged with a force in the direction of separation (denoted by the arrow D) by the grip portion 171d being gripped and pulled.

According to the second embodiment, the extracting cartridge 150 includes the cartridge base 151 containing PE (polyethylene) and PP (polypropylene) and is detachably bonded to the separator member 170 which is made of an IMX film including the PE (polyethylene) layer. This allows the cartridge base 151 to be improved in the bending strength while its temperature for thermally fusing with the separator member 170 is low and its strength of bonding with the separator member 170 remains at a level appropriate for the peeling action, as compared with a cartridge base 151 made of only PE (polyethylene) material.

According to the second embodiment, the extracting cartridge 150 includes the cartridge base 151 containing PE (polyethylene) and PP (polypropylene) and is detachably bonded to the housing member 180 which is made of an IMX film including the PE (polyethylene) layer. This allows the cartridge base 151 to be improved in the bending strength while its temperature for thermally fusing with the housing member 180 is low and its strength of boding with the housing member 180 remains at a level appropriate for the peeling action, as compared with a cartridge base 151 made of only PE (polyethylene) material.

A comparison test for examining the advantages of the second embodiment will now be explained. For comparing in the fusing temperature, the bonding strength, the bending strength, and the barrier property with the second embodiment where the ratio between PE (polyethylene) and PP (polypropylene) in the cartridge base 151 is 1:1, the comparison test prepares Comparison 1 where the cartridge base 151 is made of only PE (polyethylene), Comparison 2 where the cartridge base is made of only PP (polypropylene) , and Comparison 3 where the cartridge base is made of only PS (polystyrene). The result of the comparison test is shown in Fig. 45. As shown in Fig. 45, the fusing temperature is a temperature required for thermally fusing and bonding the IMX film to the cartridge base. The bonding strength is a strength required for separating the IMX film from the cartridge base. The bending strength is a mechanical strength for resisting the bending of the cartridge base. The barrier property is a weight of water escaped from the enclosed space defined by the cartridge base and the IMX film bonded to the cartridge base after left intact at a temperature of 60°C for one week.

Referring to Fig. 45, the temperature required for thermally fusing and bonding the IMX film to the cartridge base 151 in the second embodiment was 100°C. The temperature required for thermally fusing and bonding the IMX film to the cartridge base of Comparison 1 was 100°C. The temperature was as high as 160°C for thermally fusing and bonding the IMX film to the cartridge base of each of Comparisons 2 and 3. It is thus found from the result that the cartridge base 151 in the second embodiment made of a combination of PE (polyethylene) and PP (polypropylene) is lower in the fusing temperature than the cartridge base of Comparison 2 made of PP (polypropylene) alone and the cartridge base of Comparison 3 made of PS (polystyrene) alone while substantially equal (at 100°C of the fusing temperature) to the cartridge base of Comparison 1 made of PE (polyethylene) alone.

Also, the strength required for separating the IMX film from the cartridge base 151 in the second embodiment was 100 g. While the strength required for separating the IMX film from the cartridge base of Comparison 1 was equal to 100 g, the strength required for separating the IMX film from the cartridge base of each of Comparisons 2 and 3 was as great as 700 g. It is thus found from the result that the cartridge base 151 in the second embodiment made of a combination of PE and PP is lower in the strength for separation than the cartridge base of Comparison 2 made of PP alone and the cartridge base of Comparison 3 made of PS alone while substantially equal (at 100 g) to the cartridge base of Comparison 1 made of PE alone.

The cartridge base 151 in the second embodiment has a bending strength of 300 kg/cm$^2$. The bending strength of the cartridge base is 100 kg/cm$^2$ in Comparison 1, 500 kg/cm$^2$ in Comparison 2, and 800 kg/cm$^2$ in Comparison 3. It is also found from the result that the cartridge base 151 in the second embodiment made of a combination of PE and PP is smaller in the bending strength than the cartridge base of Comparison 2 made of PP alone and the cartridge base of Comparison 3 made of PS alone while greater in the bending strength than the cartridge base of Comparison 1 made of PE alone.

The cartridge base 151 in the second embodiment permitted as small as 1 mg of water to escape from the enclosed space defined by the cartridge base 151 and the IMX film fused to the cartridge base 151 after left intact at a temperature of 60°C for one week. While the cartridge base of each of Comparisons 1 and 2 permitted substantially 1 mg of water to escape from the enclosed space defined by the cartridge base and the IMX film bonded to the cartridge base after left intact at a temperature of 60°C for one week, the cartridge base of Comparison 3 permitted 15 mg of water to escape from the enclosed space defined by the cartridge base and the IMX film fused to the cartridge base after left intact at a temperature of 60°C for one week. It is thus found from the above result that the cartridge base 151 in the second embodiment made of a combination of PE and PP is less susceptible to water intruding the enclose space defined between the cartridge base and the IMX film fused thereto than the cartridge base of Comparison 3 made of PS alone and substantially equal (in the barrier property) to the cartridge base of Comparison 1 made of PE alone or the cartridge base of Comparison 2 made of PP alone.

As apparent from the foregoing results, the cartridge base 151 in the second embodiment made of PE (polyethylene) and PP (polypropylene) mixed at a ratio of 1:1, shown in Fig. 45, is low in the fusing temperature (at 100°) and highly favorable in the bonding strength (100 g) for separating the IMX film from the cartridge base 151 and in the barrier property as equal to the cartridge base of Comparison 1 made of PE (polyethylene) alone. It is however found that the cartridge base 151 in the second embodiment can be improved in the bending strength than the cartridge base of Comparison 1 made of PE alone.

It would be understood that the foregoing embodiments are simply illustrative but not of limitation. The present invention resurfaces not in the above description but the appended claims and is intended to cover all changes and modifications without departing from the scope of the claims.

For example, the liquid distributor member in the foregoing embodiments is made of absorbent cotton (cut cotton) as an unwoven fabric. The liquid distributor member in the present invention is not limited to the absorbent cotton (cut cotton) but may be selected from any other materials which can controllably distribute a precise amount of liquid upon coming into direct contact with the mesh sheet (a liquid retaining material) including unwoven fabrics and other than woven fabrics. The materials of the liquid distributor member may be sponge, paper, filter paper, and gel.

The liquid distributor member in the embodiments is preliminarily saturated with pure water but is not intended to be so limited. Alternatively, the liquid distributor member may be impregnated with pure water at the time of use of the extracting cartridge thus to distribute a predetermined amount of pure water upon coming in direct contact with the mesh sheet.

The liquid distributor member in the embodiments is arranged to distribute a predetermined amount of pure water upon coming in direct contact with the mesh sheet but not intended to be so limited. Alternatively, the liquid distributor member may distribute an amount of pure water via a medium such as dried absorbent cotton to the mesh sheet without coming into direct contact with the mesh sheet.

The mesh sheet in the embodiments is kept in its dry condition before the extracting cartridge is in use but not intended to be so limited. Alternatively, the mesh sheet may be impregnated with a predetermined amount of pure water in advance. Even when the mesh sheet has been dried with pure water evaporated out, it can receive a fresh supply (replenishment) of pure water from the liquid distributor member at the use of the extracting cartridge.

The extracting cartridge in the embodiments is loaded to the blood sugar level measuring apparatus when its mesh sheet has been supplied with pure water from the liquid distributor member but is not considered to be so limited. Alternatively, after the extracting cartridge is loaded to the blood sugar level measuring apparatus, its mesh sheet may be supplied with pure water from the liquid distributor member

The extracting cartridge and the liquid distributor member (absorbent cotton) in the first embodiment are accommodated together in the support member but not intended to be so limited. Alternatively, the extracting cartridge and the liquid distributor member (absorbent cotton) may be accommodated in two different support members respectively.

The extracting cartridge and the liquid distributor member (absorbent cotton) in the first embodiment are accommodated in the support member of a flexible sheet form but not intended to be so limited. Alternatively, the extracting cartridge and the liquid distributor member may be accommodated in the support member of a non-flexible box like form.

The separator member in the first embodiment is made of a flexible sheet material but not intended to be so limited. Alternatively, the separator member may be made of anon-flexible sheet material. The separator member may also be arranged not to be detachably joined to the support member.

The separator member in the first embodiment is arranged to be separated from the support member when being pulled at its grip but not considered to be so limited. The separator member may have an extension thereof extending from the support member and provided with a through hole in which the finger of a subj ect is inserted for pulling and separating the separator member from the support member. Alternatively, the separator member may have an annular member mounted thereon in which the finger of a subject is inserted for pulling out the separator member. This permits the separator member to be readily separated from the support member using the single finger.

The cartridge holder and the liquid distributor holder in the first embodiment are respectively arranged with their recessed portions shaped of a linear home base configuration so as to easily separate the separator member from the support member by starting from the corner of each recessed portion but not intended to be so limited. The recessed portions of the cartridge holder and the liquid distributor holder from which the peel-off action starts for separating the separator member from the support member may be shaped of an arcuate configuration. Alternatively, the recessed portions of the cartridge holder and the liquid distributor holder may be shaped of a circular or square configuration.

The sensor member for detecting glucose in the first embodiment is, but not limited to, an optical sensor. Alternatively, the sensor member may be an electric sensor such as a sensor electrode assembly disclosed in International Patent No. WO96/00110.

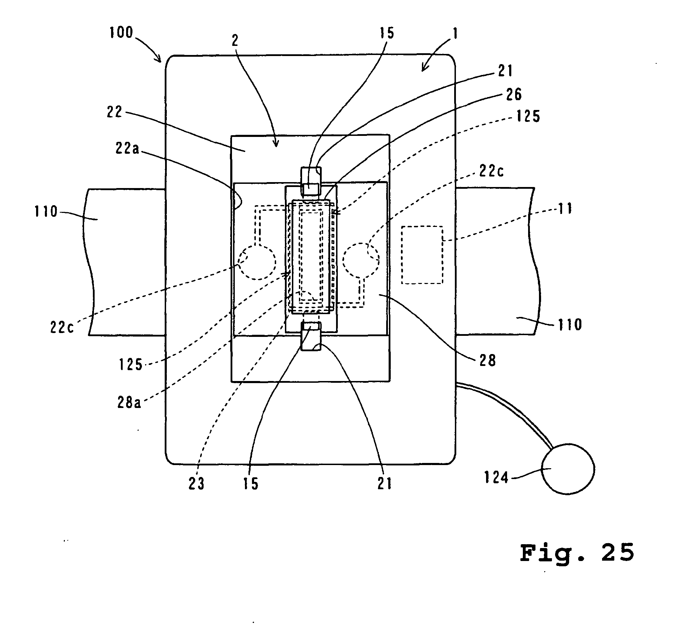

The extracting cartridge 2 in the first embodiment includes the anode 24 connected to the positive port of the constant voltage source 13 and the cathode 25 connected to the negative port of the constant voltage source 13 in the blood sugar level measuring apparatus 100, as shown in Fig. 2, but not intended to be so limited. Alternatively, the extracting cartridge 2 may have a pair of cathodes 125 connected to the negative port of the constant voltage source 13 in the blood sugar level measuring apparatus 100, such as shown in Fig. 25. Simultaneously, the positive port of the constant voltage source 13 in the blood sugar level measuring apparatus 100 is electrically connected with an anode 124 which is placed at another location of the skin of a test subject. At the case, the pure water may preferably be replaced by another electrically conductive liquid such as physiological saline.

The extracting cartridge in the first embodiment is arranged to receive a voltage from the constant voltage source but not intended to be so limited. The constant voltage source may be replaced by a constant current source. Alternatively, the extraction of glucose may be carried out using not the constant voltage source but another technique such as natural extraction, ultrasonic wave extraction, or negative pressure extraction.

The mesh sheet in the first embodiment is made of a mesh construction of nylon material as the liquid retaining material according to the present invention but not intended to be so limited. The mesh sheet may be made of any insulating material other than nylon, such as paper or resin. The liquid retaining member according to the present invention may be made of a porous sheet material of which the multiplicity of pores is produced by the action of a laser.

The constant voltage source in the first embodiment is arranged of a direct-current type for applying a voltage between the anode and the cathode but not intended to be so limited. Alternatively, the constant voltage source of an alternative current type may be used for applying a voltage between the anode and the cathode.

The present invention is described in the form of, but not limited to, the first embodiment applicable to a blood sugar level measuring apparatus for measuring an extraction of glucose from a living body. The present invention may successfully be applied to any other analyte extracting apparatus for extracting any target analyte to be analyzed other than glucose. The target analyte to be analyzed by the analyte extracting apparatus according to the present invention may be a biochemical composition or medical agent applied to a subject to be examined. Examples of the biochemical composition may include a group of proteins including albumin, globulin, and enzyme. Also, other examples than proteins of the biochemical composition are creatinine, creatine, uric acid, amino acid, fructose, galactose, pentose, glycogen, lactic acid, pyruvic acid, and ketone body. The pharmaceutical agents may include digitalis, theophylline, cardiac dysrhythmia agents, antiepileptics, aminogycoside antibiotics, glycopeptide antibiotics, antithrombus, and immunosuppressants.

When the blood sugar level measuring apparatus is combined with an analyte extracting apparatus for extracting any other object to be analyzed than glucose from a living body, its detector or controller may be configured for analyzing proteins or biochemical or medical compositions other than proteins using a method, such as HPLC (high performance liquid chromatography) different from the method described above.

The desiccator in the second embodiment is arranged of, but not limited to, a cubic shape. Alternatively, the desiccator in the present invention may be made of a sheet material.

The desiccator in the second embodiment is located between the other surface at the sensor member surface of the cartridge base and the bonding surface of the separator member but not intended to be so limited. Alternatively, the desiccator may be located between the one surface at the mesh sheet surface of the mesh sheet and the bonding surface of the separator member.

The housing member in the second embodiment is arranged for being loaded to the blood sugar level measuring apparatus after separating from the cartridge base in the extracting cartridge but not intended to be so limited. Alternatively, the housing member may be separated from the cartridge base in the extracting cartridge at the time when or after the extracting cartridge is loaded to the blood sugar level measuring apparatus.

The housing member in the second embodiment is arranged detachable from the grip of the cartridge base but not to be so limited. Alternatively, the housing member may be arranged to be separated from the cartridge base when the grip is removed from the cartridge base.

The separator member in the second embodiment is composed of two bonding laminations for having the bonding portion bonded with the housing member and the bonding portion bonded with the cartridge base but not intended to be so limited. Alternatively, the separator member may be constructed by a single lamination for having the bonding surface bonded with the housing member and the bonding surface bonded with the cartridge base. More specifically, the single lamination incorporates a five-layer construction including a PET (polyethylene terephthalate) layer, an aluminum foil, a PE (polyethylene) layer, another aluminum foil, and another PET (polyethylene terephthalate) layer arranged in this order. For having the bonding surface bonded with the housing member and the bonding surface bonded with the cartridge base, the five-layer construction is arranged separable between one group of the PET layer, the aluminum foil, and the PE layer and another group of the PE layer, the aluminum foil, and the PET layer.

The bonding surface of the separator member, the bonding surface of the cartridge base, and the housing member bonding surface of the housing member in the second embodiment are arranged to have a hexagonal shape in the plan view but not considered to be so limited. Alternatively, the bonding surface of the separator member, the bonding surface of the cartridge base, and the housing member bonding surface of the housing member may all be modified to an oval shape in the plan view whereby the peel-off action can start from one end of the oval shape opposite to the separating direction.

As described above, each embodiment of the present invention provides an analyte extracting cartridge set which can be stored for a longer period of time while being much easier in the handling and a method of analyzing with a cartridge which is improved in the storage and handling condition.

The foregoing detailed description and examples have been provided by way of explanation and illustration, and are not intended to limit the scope of the appended claims. Many variations in the presently desirable embodiments illustrated herein will be obvious to one of ordinary skill in the art, and remain within the scope of the appended claims and their equivalents.

**Claims**

1. A method for loading an analyte extracting cartridge (2; 150) onto an analyzing device (1) which is arranged to extract an analyte from a subject to be examined and analyze the analyte, comprising steps of:

   (a) supplying a liquid from a liquid distributor member (60; 160) which retains the liquid to a liquid retaining member (23) in the analyte extracting cartridge (2; 150) which can retain the liquid for holding the analyte extracted from the subject, the analyte extracting cartridge (2; 150) and the liquid distributor member (60; 160) being held by a holding member (40);
   (b) separating the analyte extracting cartridge (2; 150) from the liquid distributor member (60; 160) and holding member (40) after distributing the liquid to the liquid retaining member (23); and
   (c) loading the analyte extracting cartridge (2; 150) onto the analyzing device (1).

2. An analyte extracting cartridge set comprising:

   an analyte extracting cartridge (2; 150) adapted to be loaded to an analyzing device (1) for extracting an analyte from a subject to be examined and analyzing the analyte, the analyte extracting cartridge (2; 150) including a liquid retaining member (23) adapted to retain a liquid to hold the analyte extracted from the subject;
   a liquid distributor member (60; 160) adapted to retain the liquid and distribute the liquid to the liquid retaining member (23), wherein the liquid distributor member (60; 160) is adapted to be separated from the analyte extracting cartridge, so that it may be separated therefrom after distributing the liquid to the liquid retaining member (23);
   a holding member (40) adapted to hold the analyte extracting cartridge (2; 150) and the liquid distributor member (60; 160), the analyte extracting cartridge being detachably bonded to the holding member (40); and
   a separator member (70; 170) adapted to isolate the liquid retaining member (23) in the analyte extracting cartridge from the liquid distributor member (60; 160).

3. The analyte extracting cartridge set of claim 2, wherein
   the analyte extracting cartridge (2; 150) and the liquid distributor member (60; 160) are held by the holding member (40) so that the liquid retaining member (23) in the analyte extracting cartridge (2; 150) and the liquid distributor member (60; 160) locate opposite to each other with the separator member (70; 170) put therebetween.

4. The analyte extracting cartridge set of claim 2, wherein
   the holding member (40) further comprises a housing member (180) for housing the liquid distributor member(160), the separator member (170) is detachably bonded to both the housing member (180) and the analyte extracting cartridge (150), and
   the analyte extracting cartridge (150) and the liquid distributor member (160) are held by the holding member (40) so as to allow the liquid distributor member to come into contact with the liquid retaining member (23) when the separator member (170) is detached from the housing member (180) and the analyte extracting cartridge (150).

5. The analyte extracting cartridge set of claim 4, wherein
   the housing member (180) has a first inner surface, and
   the separator member (170) has a first surface thereof bonded to the first inner surface of the housing member and a second surface thereof bonded to the analyte extracting cartridge (150).

6. The analyte extracting cartridge set of claim 5, wherein
   the first surface of the separator member (170) has a first bonding portion detachably bonded with the first inner surface of the housing member (180),
the second surface of the separator member (170) has a second bonding portion detachably bonded with one surface of the analyte extracting cartridge (150), and
the separator member (170) has a third surface thereof, the third surface having a third bonding portion detachably bonded with the other surface of the analyte extracting cartridge (150).

**7.** The analyte extracting cartridge set of claim 5, wherein
the liquid distributor member (160) is bonded to the first inner surface (180a) of the housing member (180),
the analyte extracting cartridge (150) has a first portion thereof bonded to the second bonding portion of the separator member (170) and a second portion thereof located adjacent to the first portion,
a part of the housing member (180) is bonded with the second portion of the analyte extracting cartridge (150), and
the part of housing member (180) remains bonded with the second portion of the analyte extracting cartridge (150) when the separator member (170) has been detached.

**8.** The analyte extracting cartridge set of claim 4, wherein
the housing member (180) has a housing portion (190) for housing the liquid distributor member (160) and a cartridge bonding surface thereof detachably bonded to the analyte extracting cartridge (150).

**9.** The analyte extracting cartridge set of claim 4, wherein
the analyte extracting cartridge (150) has a cartridge grip portion (151a) for being gripped when the separator member (170) is detached from the analyte extracting cartridge (150) .

**10.** The analyte extracting cartridge set of claim 4, wherein the separator member (170) comprises:

a first bonding portion (171e) detachably bonded to the housing member (180);
a separator portion (171, 172) for isolating the liquid distributor member (160) from the liquid retaining member (23) in the analyte extracting cartridge (150);
a second bonding portion (172c) detachably bonded to the one surface (151b) of the analyte extracting cartridge (150); and
a third bonding portion (171f) detachably bonded to the other surface (151c) of the analyte extracting cartridge (150) .

**11.** The analyte extracting cartridge set of claim 10, wherein the separator member (170) comprises:

a first bonding member (171) comprising the first bonding portion (171e), the third bonding portion (171f), and a first separator portion (171b) of the separator portion; and
a second bonding member (172) comprising the second bonding portion (172c) and a second separator portion of the separator portion bonded to the first separator portion of the first bonding member (171),
wherein the first bonding portion (171e), the second bonding portion (172c), and the third bonding portion (171f) are arranged to be detached gradually from one end opposite to the direction of detaching the separator member by a force urged in the direction of separating the separator member (170).

**12.** The analyte extracting cartridge set of claim 4, wherein
the separator member (170) further comprises a separator member grip portion (171d) for being gripped when the separator member (170) is detached from the analyte extracting cartridge (150).

**13.** The analyte extracting cartridge set of claim 2, wherein
the separator member (70; 170) comprises a film made of polyethylene, and
the analyte extracting cartridge (2; 150) comprises a cartridge base detachably bonded to the separator member (70; 170) and contains polyethylene and polypropylene.

**14.** The analyte extracting cartridge set of claim 4, wherein
the housing member (180) comprises a film made of polyethylene, and
the analyte extracting cartridge (150) comprises a cartridge base detachably bonded to the housing member (180) and contains polyethylene and polypropylene.

**15.** The analyte extracting cartridge set of claim 2, wherein
the liquid retaining member (23) is made of a sheet material having a porous structure.

**16.** The analyte extracting cartridge set of claim 2, wherein
the liquid distributor member (60; 160) is arranged capable of absorbing and retaining the liquid and when coming into contact with the liquid retaining member (23), distributing a predetermined amount of the liquid to the liquid retaining member (23).

**17.** The analyte extracting cartridge set of claim 2, wherein the liquid distributed from the liquid distributor member (60; 160) is pure water or physiological saline.

**18.** The analyte extracting cartridge set of claim 2, wherein
the analyzing device (1) comprises a power source (13) for providing a skin of the subject with an electric field, and the analyte extracting cartridge (2; 150) comprises electrodes (24; 25) arranged to be connectable with the power source (13).

**19.** The analyte extracting cartridge set of claim 2, wherein
the holding member is arranged to hold the analyte extracting cartridge (2; 150) and the liquid distributor member (160) with the liquid retaining member (23) remaining in its dry condition.

**20.** The analyte extracting cartridge set of claim 2, wherein
the holding member has an inner surface facing the surface of the analyte extracting cartridge (150) for holding the liquid distributor member (160), and wherein
the separator member (170) comprises:

a first film member (172) detachably bonded to a surface of the analyte extracting cartridge (150);
a second film member (171) detachably bonded to the inner surface of the holding member defining a space with the holding member for holding the liquid distributor member (160); and
a joining member joined to one end of the first film member (172) and one end of the second film member (171), wherein
when the joining member is urged in one direction by a force, the first film member (172) and the second film member (171) are detached from the surface of the analyte extracting cartridge (150) and the inner surface of the holding member respectively.

## Patentansprüche

**1.** Ein Verfahren zum Einlegen einer Analyt-Extrahierungskassette (2; 150) in eine Analysevorrichtung (1), die zum Extrahieren eines Analyts von einem zu untersuchenden Untersuchungsobjekt und zum Analysieren des Analyts aufgebaut ist, umfassend die Schritte:

(a) Zuführen einer Flüssigkeit von einem Flüssigkeitsverteilungsteil (60; 160), das die Flüssigkeit speichert, zu einem Flüssigkeitsspeicherteil (23) in der Analyt-Extrahierungskassette (2; 150), das die Flüssigkeit zum Aufnehmen des von dem Untersuchungsobjekt extrahierten Analyts speichern kann, wobei die Analyt-Extrahierungskassette (2; 150) und das Flüssigkeitsverteilungsteil (60; 160) durch ein Aufnahmeteil (40) aufgenommen werden;
(b) Trennen der Analyt-Extrahierungskassette (2; 150) von dem Flüssigkeitsverteilungsteil (60; 160) und dem Aufnahmeteil (40) nach dem Verteilen der Flüssigkeit zu dem Flüssigkeitsspeicherteil (23); und
(c) Einlegen der Analyt-Extrahierungskassette (2; 150) in die Analysevorrichtung (1).

**2.** Analyt-Extrahierungskassettenset, umfassend:

eine Analyt-Extrahierungskassette (2; 150), die angepasst ist, um, zum Extrahieren eines Analyts von einem zu untersuchenden Untersuchungsobjekt und zum Analysieren des Analyts, in eine Analysevorrichtung (1) eingelegt zu werden, wobei die Analyt-Extrahierungskassette (2; 150) ein Flüssigkeitsspeicherteil (23) einschließt, das angepasst ist, um eine Flüssigkeit zum Aufnehmen des von dem Untersuchungsobjekt extrahierten Analyts zu speichern;
ein Flüssigkeitsverteilungsteil (60; 160), das angepasst ist, um die Flüssigkeit zu speichern und die Flüssigkeit an das Flüssigkeitsspeicherteil (23) zu verteilen, wobei das Flüssigkeitsverteilungsteil (60; 160) angepasst ist, um von der Analyt-Extrahierungskassette getrennt zu werden, so dass es von dieser nach Verteilung der Flüssigkeit zu dem Flüssigkeitsspeicherteil (23) getrennt werden kann;

ein Aufnahmeteil (40), angepasst, um die Analyt-Extrahierungskassette (2; 150) und das Flüssigkeitsverteilungsteil (60; 160) aufzunehmen, wobei die Analyt-Extrahierungskassette abnehmbar mit dem Aufnahmeteil (40) verbunden ist; und

ein Trennteil (70; 170), angepasst, um das Flüssigkeitsspeicherteil (23) in der Analyt-Extrahierungskassette von dem Flüssigkeitsverteilungsteil (60; 160) zu isolieren.

3. Analyt-Extrahierungskassettenset gemäß Anspruch 2,' bei dem
die Analyt-Extrahierungskassette (2; 150) und das Flüssigkeitsverteilungsteil (60; 160) durch das Aufnahmeteil (40) aufgenommen werden, so dass das Flüssigkeitsspeicherteil (23) in der Analyt-Extrahierungskassette (2; 150) und das Flüssigkeitsverteilungsteil (60; 160) einander gegenüberliegend mit einem dazwischen gelegten Trennteil (70; 170) angeordnet sind.

4. Analyt-Extrahierungskassettenset gemäß Anspruch 2, bei dem
das Aufnahmeteil (40) weiterhin ein Gehäuseteil (180) zum Unterbringen des Flüssigkeitsverteilungsteils (160) aufweist,
wobei das Trennteil (170) abnehmbar mit sowohl dem Gehäuseteil (180) als auch der Analyt-Extrahierungskassette (150) verbunden ist, und
die Analyt-Extrahierungskassette (150) und das Flüssigkeitsverteilungsteil (160) durch das Aufnahmeteil (40) aufgenommen werden, um dem Flüssigkeitsverteilungsteil zu erlauben mit dem Flüssigkeitsspeicherteil (23) in Kontakt zu kommen, wenn das Trennteil (170) von dem Gehäuseteil (180) und der Analyt-Extrahierungskassette (150) abgenommen wird.

5. Analyt-Extrahierungskassettenset gemäß Anspruch 4, bei dem
das Gehäuseteil (180) eine erste innere Oberfläche aufweist, und
das Trennteil (170) eine erste Oberfläche aufweist, die mit der ersten inneren Oberfläche des Gehäuseteils verbunden ist und eine zweite Oberfläche, die mit der Analyt-Extrahierungskassette (150) verbunden ist.

6. Analyt-Extrahierungskassettenset gemäß Anspruch 5, bei dem
die erste Oberfläche des Trennteils (170) einen ersten Verbindungsabschnitt aufweist, der mit der ersten inneren Oberfläche des Gehäuseteils (180) abnehmbar verbunden ist,
die zweite Oberfläche des Trennteils (170) einen zweiten Verbindungsabschnitt aufweist, der mit einer Oberfläche der Analyt-Extrahierungskassette (150) abnehmbar verbunden ist, und
das Trennteil (170) eine dritte Oberfläche aufweist, wobei die dritte Oberfläche einen dritten Verbindungsabschnitt aufweist, der abnehmbar mit der anderen Oberfläche der Analyt-Extrahierungskassette (150) verbunden ist.

7. Analyt-Extrahierungskassettenset gemäß Anspruch 5, bei dem
das Flüssigkeitsverteilungsteil (160) mit der ersten inneren Oberfläche (180a) des Gehäuseteils (180) verbunden ist, wobei die Analyt-Extrahierungskassette (150) einen ersten Abschnitt aufweist, verbunden mit dem zweiten Verbindungsabschnitt des Trennteils (170) und einen zweiten Abschnitt, angrenzend an den ersten Abschnitt angeordnet,
ein Teil des Gehäuseteils (180) mit dem zweiten Abschnitt der Analyt-Extrahierungskassette (150) verbunden ist, und
der Teil des Gehäuseteils (180) mit dem zweiten Abschnitt der Analyt-Extrahierungskassette (150) verbunden bleibt, wenn das Trennteil (170) abgenommen worden ist.

8. Analyt-Extrahierungskassettenset gemäß Anspruch 4, bei dem
das Gehäuseteil (180) einen Gehäuseabschnitt (190) zum Unterbringen des Flüssigkeitsverteilungsteils (160) aufweist und eine Kassettenverbindungsoberfläche abnehmbar mit der Analyt-Extrahierungskassette (150) verbunden ist.

9. Analyt-Extrahierungskassettenset gemäß Anspruch 4, bei dem die Analyt-Extrahierungskassette (150) einen Kassettengriffabschnitt (151a) aufweist und zwar zum Greifen wenn das Trennteil (170) von der Analyt-Extrahierungskassette (150) abgenommen wird.

10. Analyt-Extrahierungskassettenset gemäß Anspruch 4, bei dem das Trennteil (170) umfasst:

einen ersten Verbindungsabschnitt (171e), abnehmbar mit dem Gehäuseteil (180) verbunden;
einen Trennabschnitt (171, 172) zum Isolieren des Flüssigkeitsverteilungsteils (160) von dem Flüssigkeitsspeicherteil (23) in der Analyt-Extrahierungskassette (150);

einen zweiten Verbindungsabschnitt (172c), abnehmbar mit der einen Oberfläche (151b) der Analyt-Extrahierungskassette (150) verbunden; und

einen dritten Verbindungsabschnitt (171f), abnehmbar mit der anderen Oberfläche (151c) der Analyt-Extrahierungskassette (150) verbunden.

11. Analyt-Extrahierungskassettenset gemäß Anspruch 10, bei dem das Trennteil (170) umfasst:

ein erstes Verbindungsteil (171), das den ersten Verbindungsabschnitt (171e), den dritten Verbindungsabschnitt (171f) und einen ersten Trennabschnitt (171b) des Trennabschnitts aufweist; und

ein zweites Verbindungsteil (172), das den zweiten Verbindungsabschnitt (172c) und einen zweiten Trennabschnitt des Trennabschnitts aufweist, der mit dem ersten Trennabschnitt des ersten Verbindungsteils (171) verbunden ist,

wobei der erste Verbindungsabschnitt (171e), der zweite Verbindungsabschnitt (172c) und der dritte Verbindungsabschnitt (171f) aufgebaut sind, um durch eine in der Richtung zum Trennen des Trennteils (170) ausgeübte Kraft schrittweise von einem der Richtung zum Trennen des Trennteils gegenüberliegenden Ende abgenommen zu werden.

12. Analyt-Extrahierungskassettenset gemäß Anspruch 4, bei dem
das Trennteil (170) weiterhin einen Trennteilgriffabschnitt (171d) aufweist und zwar zum Greifen wenn das Trennteil (170) von der Analyt-Extrahierungskassette (150) abgenommen wird.

13. Analyt-Extrahierungskassettenset gemäß Anspruch 2, bei dem
das Trennteil (70; 170) eine aus Polyethylen hergestellte Folie aufweist, und
die Analyt-Extrahierungskassette (2; 150) einen Kassettenboden aufweist, der abnehmbar mit dem Trennteil (70; 170) verbunden ist und Polyethylen und Polypropylen enthält.

14. Analyt-Extrahierungskassettenset gemäß Anspruch 4, bei dem
das Gehäuseteil (180) eine aus Polyethylen hergestellte Folie aufweist, und
die Analyt-Extrahierungskassette (150) einen Kassettenboden aufweist, der abnehmbar mit dem Gehäuseteil (180) verbunden ist und Polyethylen und Polypropylen enthält.

15. Analyt-Extrahierungskassettenset gemäß Anspruch 2, bei dem
das Flüssigkeitsspeicherteil (23) aus einem Sheet-Material hergestellt ist, das eine poröse Struktur aufweist.

16. Analyt-Extrahierungskassettenset gemäß Anspruch 2, bei dem
das Flüssigkeitsverteilungsteil (60; 160) aufgebaut ist, um in der Lage zu sein, die Flüssigkeit zu absorbieren und zu speichern und wenn es mit dem Flüssigkeitsspeicherteil (23) in Kontakt kommt, eine festgelegte Menge der Flüssigkeit an das Flüssigkeitsspeicherteil (23) zu verteilen.

17. Analyt-Extrahierungskassettenset gemäß Anspruch 2, bei dem
die von dem Flüssigkeitsverteilungsteil (60; 160) verteilte Flüssigkeit reines Wasser oder physiologische Kochsalzlösung ist.

18. Analyt-Extrahierungskassettenset gemäß Anspruch 2, bei dem
die Analysevorrichtung (1) eine Spannungsquelle (13) zum Versorgen einer Haut des Untersuchungsobjekts mit einem elektrischen Feld aufweist und
die Analyt-Extrahierungskassette (2; 150) Elektroden (24; 25) aufweist, die verbindbar mit der Spannungsquelle (13) aufgebaut sind.

19. Analyt-Extrahierungskassettenset gemäß Anspruch 2, bei dem
das Aufnahmeteil aufgebaut ist, um die Analyt-Extrahierungskassette (2; 150) und das Flüssigkeitsverteilungsteil (160) mit dem Flüssigkeitsspeicherteil (23), das seinen trockenen Zustand beibehält, aufzunehmen.

20. Analyt-Extrahierungskassettenset gemäß Anspruch 2, bei dem
das Aufnahmeteil eine innere Oberfläche aufweist, die der Oberfläche der Analyt-Extrahierungskassette (150) zum Aufnehmen des Flüssigkeitsverteilungsteils (160) gegenüberliegt und wobei
das Trennteil (170) aufweist:

EP 1 683 476 B1

ein erstes Folienteil (172), abnehmbar mit einer Oberfläche der Analyt-Extrahierungskassette (150) verbunden;
ein zweites Folienteil (171), abnehmbar mit der inneren Oberfläche des Aufnahmeteils verbunden, das einen Bereich mit dem Aufnahmeteil zum Aufnehmen des Flüssigkeitsverteilungsteils (160) definiert; und
ein Verbindungsteil, verbunden mit einem Ende des ersten Folienteils (172) und einem Ende des zweiten Folienteils (171), wobei
wenn das Verbindungsteil durch eine Kraft in eine Richtung gedrängt wird, das erste Folienteil (172) und das zweite Folienteil (171) jeweils von der Oberfläche der Analyt-Extrahierungskassette (150) und der inneren Oberfläche des Aufnahmeteils abgenommen werden.

## Revendications

1. Procédé pour charger une cartouche d'extraction de substance à analyser (2 ; 150) sur un dispositif d'analyse (1) qui est agencé pour extraire une substance à analyser d'un sujet à examiner et analyser la substance à analyser, comprenant les étapes consistant à :

   (a) fournir un liquide provenant d'un élément distributeur de liquide (60 ; 160) qui retient le liquide dans un élément de retenue de liquide (23) dans la cartouche d'extraction de substance à analyser (2 ; 150) qui peut retenir le liquide pour maintenir la substance à analyser extraite du sujet, la cartouche d'extraction de substance à analyser (2 ; 150) et l'élément distributeur de liquide (60 ; 160) étant maintenus par un élément de maintien (40) ;
   (b) séparer la cartouche d'extraction de substance à analyser (2 ; 150) depuis l'élément distributeur de liquide (60 ; 160) et l'élément de maintien (40) après distribution du liquide à l'élément de retenue de liquide (23) ; et
   (c) charger la cartouche d'extraction de substance à analyser (2 ; 150) sur le dispositif d'analyse (1).

2. Ensemble formant cartouche d'extraction de substance à analyser comprenant :

   une cartouche d'extraction de substance à analyser (2 ; 150) conçue pour être chargée sur un dispositif d'analyse (1) pour extraire une substance à analyser d'un sujet à examiner et pour analyser la substance à analyser, la cartouche d'extraction de substance à analyser (2 ; 150) incluant un élément de retenue de liquide (23) conçu pour retenir un liquide pour maintenir la substance à analyser extraite du sujet ;
   un élément distributeur de liquide (60 ; 160) conçu pour retenir le liquide et distribuer le liquide à l'élément de retenue de liquide (23), dans lequel l'élément distributeur de liquide (60 ; 160) est conçu pour être séparé de la cartouche d'extraction de substance à analyser, de sorte qu'il peut être séparé de celle-ci après distribution du liquide à l'élément de retenue de liquide (23) ;
   un élément de maintien (40) conçu pour maintenir la cartouche d'extraction de substance à analyser (2 ; 150) et l'élément distributeur de liquide (60 ; 160), la cartouche d'extraction de substance à analyser étant liée de façon amovible à l'élément de maintien (40) ; et
   un élément séparateur (70 ; 170) conçu pour isoler l'élément de retenue de liquide (23) dans la cartouche d'extraction de substance à analyser de l'élément distributeur de liquide (60 ; 160).

3. Ensemble formant cartouche d'extraction de substance à analyser selon la revendication 2, dans lequel la cartouche d'extraction de substance à analyser (2 ; 150) et l'élément distributeur de liquide (60 ; 160) sont maintenus par l'élément de maintien (40) de sorte que l'élément de retenue de liquide {23) dans la cartouche d'extraction de substance à analyser (2 ; 150) et l'élément distributeur de liquide (60 ; 160) se situent l'un en face de l'autre, l'élément séparateur (70 ; 170) étant positionné entre ceux-ci.

4. Ensemble formant cartouche d'extraction de substance à analyser selon la revendication 2, dans lequel l'élément de maintien (40) comprend en outre un élément de logement (180) pour loger l'élément distributeur de liquide (160),
   l'élément séparateur (170) est lié de façon amovible tant à l'élément de logement (180) qu'à la cartouche d'extraction de substance à analyser (150), et
   la cartouche d'extraction de substance à analyser (150) et l'élément distributeur de liquide (160) sont maintenus par l'élément de maintien (40) de façon à permettre à l'élément distributeur de liquide d'entrer en contact avec l'élément de retenue de liquide (23) quand l'élément séparateur (170) est détaché de l'élément de logement (180) et de la cartouche d'extraction de substance à analyser (150).

5. Ensemble formant cartouche d'extraction de substance à analyser selon la revendication 4, dans lequel l'élément de logement (180) a une première surface intérieure, et

23

l'élément séparateur (170) a une première surface de celui-ci liée à la première surface intérieure de l'élément de logement et une deuxième surface de celui-ci liée à la cartouche d'extraction de substance à analyser (150).

6. Ensemble formant cartouche d'extraction de substance à analyser selon la revendication 5, dans lequel
la première surface de l'élément séparateur (170) a une première partie de liaison liée de façon amovible à la première surface intérieure de l'élément de logement (180),
la deuxième surface de l'élément séparateur (170) a une deuxième partie de liaison liée de façon amovible à une surface de la cartouche d'extraction de substance à analyser (150), et
l'élément séparateur (170) a une troisième surface de celui-ci, la troisième surface ayant une troisième partie de liaison liée de façon amovible à l'autre surface de la cartouche d'extraction de substance à analyser (150).

7. Ensemble formant cartouche d'extraction de substance à analyser selon la revendication 5, dans lequel
l'élément distributeur de liquide (160) est lié à la première surface intérieure (180a) de l'élément de logement (180),
la cartouche d'extraction de substance à analyser (150) a une première partie de celle-ci liée à la deuxième partie de liaison de l'élément séparateur (170) et une seconde partie de celle-ci située adjacente à la première partie,
une partie de l'élément de logement (180) est liée à la seconde partie de la cartouche d'extraction de substance à analyser (150), et
la partie de l'élément de logement (180) reste liée à la seconde partie de la cartouche d'extraction de substance à analyser (150) quand l'élément séparateur (170) a été détaché.

8. Ensemble formant cartouche d'extraction de substance à analyser selon la revendication 4, dans lequel
l'élément de logement (180) a une partie de logement (190) pour loger l'élément distributeur de liquide (160) et une surface de liaison de cartouche de ce dernier liée de façon amovible à la cartouche d'extraction de substance à analyser (150).

9. Ensemble formant cartouche d'extraction de substance à analyser selon la revendication 4, dans lequel
la cartouche d'extraction de substance à analyser (150) a une partie de saisie de cartouche (151a) pour être saisie quand l'élément séparateur (170) est détaché de la cartouche d'extraction de substance à analyser (150).

10. Ensemble formant cartouche d'extraction de substance à analyser selon la revendication 4, dans lequel l'élément séparateur (170) comprend :

une première partie de liaison (171e) liée de façon amovible à l'élément de logement (180) ;
une partie séparatrice (171, 172) pour isoler l'élément distributeur de liquide (160) de l'élément de retenue de liquide (23) dans la cartouche d'extraction de substance à analyser (150) ;
une deuxième partie de liaison (172c) liée de façon amovible à la surface particulière (151b) de la cartouche d'extraction de substance à analyser (150) ; et
une troisième partie de liaison (171f) liée de façon amovible à l'autre surface (151c) de la cartouche d'extraction de substance à analyser (150).

11. Ensemble formant cartouche d'extraction de substance à analyser selon la revendication 10, dans lequel l'élément séparateur (170) comprend :

un premier élément de liaison (171) comprenant la première partie de liaison (171e), la troisième partie de liaison (171f), et une première partie séparatrice (171b) de la partie séparatrice ; et
un second élément de liaison (172) comprenant la deuxième partie de liaison (172c) et une seconde partie séparatrice de la partie séparatrice liée à la première partie séparatrice du premier élément de liaison (171),
dans lequel la première partie de liaison (171e), la deuxième partie de liaison (172c) et la troisième partie de liaison (171f) sont agencées pour être graduellement détachées d'une extrémité opposée à la direction de détachement de l'élément séparateur par une force poussée dans la direction de séparation de l'élément séparateur (170).

12. Ensemble formant cartouche d'extraction de substance à analyser selon la revendication 4, dans lequel
l'élément séparateur (170) comprend en outre une partie de saisie d'élément séparateur (171d) pour être saisie quand l'élément séparateur (170) est détaché de la cartouche d'extraction de substance à analyser (150).

13. Ensemble formant cartouche d'extraction de substance à analyser selon la revendication 2, dans lequel
l'élément séparateur (70 ; 170) comprend un film fait de polyéthylène, et

la cartouche d'extraction de substance à analyser (2 ; 150) comprend une base de cartouche liée de façon amovible à l'élément séparateur (70 ; 170) et contient du polyéthylène et du polypropylène.

14. Ensemble formant cartouche d'extraction de substance à analyser selon la revendication 4, dans lequel l'élément de logement (180) comprend un film fait de polyéthylène, et la cartouche d'extraction de substance à analyser (150) comprend une base de cartouche liée de façon amovible à l'élément de logement (180) et contient du polyéthylène et du polypropylène.

15. Ensemble formant cartouche d'extraction de substance à analyser selon la revendication 2, dans lequel l'élément de retenue de liquide (23) est fait d'une matière en feuille ayant une structure poreuse.

16. Ensemble formant cartouche d'extraction de substance à analyser selon la revendication 2, dans lequel l'élément distributeur de liquide (60 ; 160) est agencé pour être capable d'absorber et de retenir le liquide et en entrant en contact avec l'élément de retenue de liquide (23), distribuer une quantité prédéterminée du liquide à l'élément de retenue de liquide (23).

17. Ensemble formant cartouche d'extraction de substance à analyser selon la revendication 2, dans lequel le liquide distribué à partir de l'élément distributeur de liquide (60 ; 160) est de l'eau pure ou une solution saline physiologique.

18. Ensemble formant cartouche d'extraction de substance à analyser selon la revendication 2, dans lequel le dispositif d'analyse (1) comprend une source d'énergie (13) pour procurer un champ électrique à la peau du sujet, et la cartouche d'extraction de substance à analyser (2 ; 150) comprend des électrodes (24 ; 25) agencées pour pouvoir être reliées à la source d'énergie (13).

19. Ensemble formant cartouche d'extraction de substance à analyser selon la revendication 2, dans lequel l'élément de maintien est agencé pour maintenir la cartouche d'extraction de substance à analyser (2 ; 150) et l'élément distributeur de liquide (160), l'élément de retenue de liquide (23) restant dans son état sec.

20. Ensemble formant cartouche d'extraction de substance à analyser selon la revendication 2, dans lequel l'élément de maintien a une surface intérieure faisant face à la surface de la cartouche d'extraction de substance à analyser (150) pour maintenir l'élément distributeur de liquide (160), et dans lequel l'élément séparateur (170) comprend :

   un premier élément formant film (172) lié de façon amovible à une surface de la cartouche d'extraction de substance à analyser (150) ;
   un second élément formant film (171) lié de façon amovible à la surface intérieure de l'élément de maintien définissant un espace avec l'élément de maintien pour maintenir l'élément distributeur de liquide (160) ; et
   un élément de jonction joint à une extrémité du premier élément formant film (172) et une extrémité du second élément formant film (171), dans lequel
   lorsque l'élément de jonction est poussé dans une direction par une force, le premier élément formant film (172) et le second élément formant film (171) sont détachés respectivement de la surface de la cartouche d'extraction de substance à analyser (150) et de la surface intérieure de l'élément de maintien.

**Fig. 1**

**Fig. 2**

Fig. 3

Fig. 4

Fig. 5

Fig. 6

Fig. 7

Fig. 8

Fig. 9

Fig. 10

**Fig. 11**

**Fig. 12**

121  121  121  121  121  Epidermis ─

Corneal layer

Granular layer

Dermis

Subcutaneous
tissue

**Fig. 13**

Flowchart of measuring blood sugar level

```
                    ┌─────────┐
                    │  Start  │
                    └─────────┘
                         │
                         ▼
S1 ──────┐      ┌──────────────────────┐
         │      │   Mount band member   │
         │      │      to the wrist     │
                └──────────────────────┘
                         │
                         ▼
S2 ──────┐      ┌──────────────────────┐
         │      │ Take out unused extracting │
         │      │  cartridge from extracting │
         │      │      cartridge set    │
                └──────────────────────┘
                         │
                         ▼
S3 ──────┐      ┌──────────────────────┐
         │      │ Load unused extracting cartridge │
         │      │ onto blood sugar level measuring │
         │      │       apparatus       │
                └──────────────────────┘
                         │
                         ▼
S4 ──────┐      ┌──────────────────────┐
         │      │  Subject extracting area to │
         │      │ preparatory step (piecing with │
         │      │      needle roller)   │
                └──────────────────────┘
                         │
                         ▼
S5 ──────┐      ┌──────────────────────┐
         │      │ Mount blood sugar level measuring │
         │      │   apparatus to band member │
                └──────────────────────┘
                         │
                         ▼
S6 ──────┐      ┌──────────────────────┐
         │      │ Apply constant voltage (0.8 V) to │
         │      │    extract body fluid │
                └──────────────────────┘
                         │
                         ▼
S7 ──────┐      ┌──────────────────────┐
         │      │ Measure the extraction of glucose │
         │      │   level with sensor member │
                └──────────────────────┘
                         │
                         ▼
S8 ──────┐      ┌──────────────────────┐
         │      │ Calculate and display the blood │
         │      │ sugar level using Equation (1) │
                └──────────────────────┘
                         │
                         ▼
S9 ──────┐      ┌──────────────────────┐
         │      │  Dismount blood sugar level │
         │      │ measuring apparatus from band │
         │      │        member         │
                └──────────────────────┘
                         │
                         ▼
S10 ─────┐      ┌──────────────────────┐
         │      │ Unload used extracting cartridge │
         │      │ from blood sugar level measuring │
         │      │       apparatus       │
                └──────────────────────┘
                         │
                         ▼
S11 ─────┐      ┌──────────────────────┐
         │      │ Dismount band member from the │
         │      │         wrist         │
                └──────────────────────┘
                         │
                         ▼
                    ┌─────────┐
                    │   End   │
                    └─────────┘
```

**Fig. 14**

**Fig. 15**

**Fig. 16**

**Fig. 17**

34

Fig. 18

Fig. 19

Relationship between storage of pure water in
liquid distributor member and distributed amount

Distributed
amount (ml)

Storage of pure water in liquid
distributor member (ml)

Fig. 20

121  121  121  121  121

Epidermis

Corneal layer

Granular layer

Dermis

Subcutaneous
tissue

Fig. 21

121 121 121 121 121

23

Epidermis

Corneal layer

Granular layer

Dermis

Subcutaneous
tissue

**Fig. 22**

Fig. 23

121   121   121   121   121

23

Epidermis

Corneal layer

Granular layer

Dermis

Subcutaneous
tissue

**Fig. 24**

40

Fig. 25

**Fig. 26**

**Fig. 27**

Fig. 28

Fig. 29

Fig. 30

Fig. 31

200a

21
28
171c
171f
21
26
D
151e
151c
151
171
170
172
151a
180b
180a
180
151b
510e
171a
151f
172b
171b
50
150
172a

**Fig. 32**

200a

151   150   26   151c
151a
D
180   151f   180b   190   161  23   171b  172b  151b   171 172
180a   160   171a   172a   170

**Fig. 33**

**Fig. 34**

**Fig. 35**

EP 1 683 476 B1

Fig. 36

Fig. 37

47

151a   151 150   26                                              151c        200a

D

151f                                                                         171 172
180        180b   151b   160        23                   171a    180a   170
                              161                    172a

**Fig. 38**

200a

D

21
28
21   26
151e                                                      172a
151c
151                                                                    171
151a                                                                  172   170
180b                                                         171a
180                                                  180a
          510e        50        180c
151f                151b                150

**Fig. 39**

**Fig. 40**

**Fig. 41**

Fig. 42

Fig. 43

**Fig. 44**

| Material of cartridge base | Fusing temperature | Bonding strength | Bending strength | Barrier property |
|---|---|---|---|---|
| Mixture of polyethylene and polypropylene (second embodiment) | ◎ (100℃~) | ◎ (100g~) | ○ (300kg/cm²) | ◎ (1mg) |
| Polyethylene (Comparison 1) | ◎ (100℃~) | ◎ (100g~) | △ (100kg/cm²) | ◎ (1mg) |
| Polypropylene (Comparison 2) | △ (160℃~) | △ (700g~) | ○ (500kg/cm²) | ◎ (1mg) |
| Styrene (polystyrene) (Comparison 3) | △ (160℃~) | △ (700g~) | ◎ (800kg/cm²) | △ (15mg) |

◎···Excellent
○···Good
△···Fair

**Fig. 45**

**EP 1 683 476 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9600110 A
- US 6503198 B1
- WO 9702811 A